# EUROPEAN PATENT APPLICATION

(11) **EP 4 079 839 A1**
(43) Date of publication of application: **26.10.2022**
(21) Application number: 21169999.6
(22) Date of filing: 22.04.2021
(51) Int. Cl.: C12N 5/071, C12N 5/10

(54) **GENETICALLY MODIFIED RECOMBINANT CELL LINES**

(71) Applicant: Fraunhofer-Gesellschaft zur Förderung der angewandten Forschung e.V., 80686 München (DE)
(72) Inventor: NOLL, Gundula, 48145 Münster (DE); PRÜFER, Dirk, 48145 Münster (DE); KRONENBERG, Julia, 49074 Osnabrück (DE); KÄNEL, Philip, 48329 Havixbeck (DE); SCHROEDTER, Katrin, 48145 Münster (DE)
(74) Representative: Kuttenkeuler, David

(57) **Abstract**

Glycoproteins that are transgenically produced in mammalian cells exhibit non-human glycan structures. As in humans, this can possibly lead to immune responses, the drug manufacturing potential of these drugs is limited. On the other hand, recombinant protein production in human cells is inefficient due to the cells' poor protein yields, proliferation potential and cellular density. The present application solves these issues by providing a recombinant vertebrate cell that is comprising a non-vertebrate and/or artificial phosphatidylethanolamine-binding protein (PEBP). Compared to a parent cell line, the recombinant cells of the invention exhibit improved cell growth, protein yield and excellent compatibility with other established protein production methods. Furthermore, methods, for producing a cell line with improved vitality, protein expression and cell growth characteristics by introducing a non-vertebrate and/or artificial PEBP is given. Moreover, both a nucleic acid construct that is suitable for regulating recombinant protein expression in a cell by coding for such a PEBP and a recombinant cell comprising such a nucleic acid construct is provided. Lastly, a method for the recombinant expression of a target protein by culturing such a recombinant vertebrate cell of the invention is given, wherein the cell is also comprising an expression construct encoding the target protein.

## Description

### FIELD OF THE INVENTION

Glycoproteins that are transgenically produced in mammalian cells exhibit non-human glycan structures. As in humans, this can possibly lead to immune responses, the drug manufacturing potential of these drugs is limited. On the other hand, recombinant protein production in human cells is inefficient due to the cells' poor protein yields, proliferation potential and cellular density. The present application solves these issues by providing a recombinant vertebrate cell that is comprising a non-vertebrate and/or artificial phosphatidylethanolamine-binding protein (PEBP). Compared to a parent cell line, the recombinant cells of the invention exhibit improved cell growth, protein yield and excellent compatibility with other established protein production methods. Furthermore, methods, for producing a cell line with improved vitality, protein expression and cell growth characteristics by introducing a non-vertebrate and/or artificial PEBP is given. Moreover, both a nucleic acid construct that is suitable for regulating recombinant protein expression in a cell by coding for such a PEBP and a recombinant cell comprising such a nucleic acid construct is provided. Lastly, a method for the recombinant expression of a target protein by culturing such a recombinant vertebrate cell of the invention is given, wherein the cell is also comprising an expression construct encoding the target protein.

### DESCRIPTION

Today, the majority of biopharmaceuticals are produced in recombinant organisms. Bacterial expression systems, such as *E. choli,* are used to produce simple non-glycosylated monoclonal antibodies, hormones, cytokines and enzymes. Mammalian cells on the other hand are the predominant system for the production of recombinant proteins that display complex posttranslational modification. Although they are higher cost, they achieve superior protein activity, stability, and immunogenicity compared to non-mammalian bacteria and yeast hosts.

The production of biopharmaceutical proteins in mammalian cells by transient expression or stable transformation requires robust and viable cells. As of date, the most used mammalian expression systems are chinese hamster ovary (CHO), Sp2/0 and or murine myeloma (NSo) cell lines. The benefits of these cells include a high recombinant protein yield, ease of transfection, and ability to grow at high densities in large-scale bioreactors. However, these mammalian cells produce glycoproteins with non-human glycan structures such as galactose-α1,3-galactose or *N*-glycolylneuraminic acid. These structures potentially trigger immunogenic responses and affect their biological activity, protein stability and clearance rate, which necessitates extensive and costly downstream processing. For these reasons, human cell lines are increasingly used for recombinant protein production. (Chin et al., Sci Rep. 9, 16768, 2019, doi: 10.1038/S41598-019-53391-z)

The most important human cell lines for this use are PERC.6, HEK-293 and HT-1080 cells. In comparison to other mammalian cells, human cells however do not yet give the same performance regarding growth rate, cellular density and protein production rate. To improve these performance indicators, commonly used strategies include isolation and clonal expansion of promising cell lines and the introduction of viral nucleic acids. HEK-293 in particular exhibits several commercially used clonal isolates that allow for improved culturing, transfection and protein production efficiencies such as HEK-293H. Other modified HEK-293 cells include the HEK293-T and HEK-293E cell lines. HEK293-T expresses the simian virus 40 large T antigen and are capable of expressing high titers of viral gene vectors for use in gene therapy or for viral vaccines. HEK-293-E cell lines constitutively express the Epstein-Barr virus EBNA-1 gene that is controlled by the cytomegalovirus promoter and demonstrate a greater growth rate and cell density relative to parental HEK293 cells. (Dumont et al., Crit Rev Biotechnol. 36(6), 1110, 2016, doi: 10.3109/07388551.2015.1084266)

Nevertheless, the possibilities of these optimizations are limited by a narrow number of available cell culture conditions and vector combinations. Efforts to improve the efficiency of a protein production are usually based on tweaking either one of these parameters and rely on chance rather than design. Cellular engineering approaches to improve cellular protein production rely on the manipulation of cell cycle and survival regulators. However, these methods are not suitable to increase single beneficial cell properties due to interlinked signalling pathways. For example, the expression of apoptosis-inhibiting proteins can also lead to decrease in the production of target proteins.

Phosphatidylethanolamine binding proteins are a superfamily of proteins with hundreds of members, only four of which (PEBP1-4) have been documented in mammalian cells. Their numerous biological functions include the biogenesis, fluidity, and formation of functional domains in membranes, stimulation of acetylcholine secretion during neuronal development, serine protease inhibition in neuronal tissue, spermatogenesis or sperm maturation. Notably, PEBP1 and PEBP4 are also known to influence cell proliferation, differentiation and apoptosis, by acting as regulators on the PI3K/AKT/mTOR and MAPK/ERK pathways. As such, they are promising targets for host cell engineering. (He et al., Biochim Biophys Acta. 1863(7 Pt A), 1682, 2016, doi: 10.1016/j.bbamcr.2016.03.022)

Influencing the productivity of human cells by directly altering PEBP1 (also known as RKIP) and PEBP4 is difficult to implement for several reasons: Human PEBPs are intricately linked to phosphorylation-dependent signaling hubs and their manipulation can increase off-target effects such as the induction of apoptosis. The function of human and other mammalian PEBPs furthermore requires posttranslational modification by other signaling pathways. In particular, mammalian PEPBs exhibit an elongated carboxyl-terminus. This terminus is determining their binding affinity to other molecules and is subject to conformational changes by co-regulators. Plant based PEBPs on the other hand lack this feature.

Thus, it is an object of the invention to considerably improve the performance of mammalian and human cells in recombinant protein production. The toolbox of cellular engineering needs to be expanded with functional proteins that can preferably be targeted by orthogonal signal pathways. Hence, the invention seeks to provide recombinantly altered cell lines with various improved characteristics compared to their origin, in which characteristics may range from cellular protein expression, cell viability, cell vitality, cell growth and/or - proliferation, cell metabolic activity, cell signaling capacity, and many more.

### BRIEF DESCRIPTION OF THE INVENTION

The present invention addresses this problem, by providing a recombinant vertebrate cell, comprising of a nucleic acid sequence that is encoding for a non-vertebrate and/or artificial phosphatidylethanolamine-binding protein (PEBP) and/or that is comprising a protein consisting of an amino acid sequence of the non-vertebrate and/or artificial phosphatidylethanolamine-binding protein (PEBP). Furthermore, the invention pertaining to a method for producing a cell line with improved characteristics selected from vitality, protein expression and cell growth. This increased vitality allows for a fast gain of cellular mass and an easy upscaling of protein production. Furthermore, the introduction of non-vertebrate and/or artificial PEBPs gives an additional means to influence the productivity of recombinant cells, that can be manipulated independently from established methods such as the introduction of viral expression systems or optimization of culture conditions.

Generally, and by way of brief description, the main aspects of the present invention can be described as follows:
a **first aspect,** the invention pertains a recombinant vertebrate cell, comprising (i) a nucleic acid sequence encoding for a non-vertebrate and/or artificial phosphatidylethanolamine-binding protein (PEBP) and/or (ii) a protein consisting of an amino acid sequence of the non-vertebrate and/or artificial phosphatidylethanolamine-binding protein (PEBP).
In a **second aspect,** the invention pertains a method producing a cell line with improved characteristics selected from vitality, protein expression and cell growth, comprising the steps of providing a candidate vertebrate cell of a selected vertebrate species, introducing into the vertebrate cell a non-vertebrate and/or artificial phosphatidylethanolamine-binding protein (PEBP), and culturing the cell to obtain the cell line with improved characteristic.
In a **third aspect,** the invention pertains to an isolated nucleic acid construct suitable for recombinant expression in a vertebrate cell or cell line, comprising a coding element operatively linked with one or more expression elements which is suitable for regulating protein expression in the vertebrate cell, wherein the coding element comprises a nucleic acid sequence encoding a non-vertebrate and/or artificial phosphatidylethanolamine-binding protein (PEBP).
In a **fourth aspect,** the invention pertains to a recombinant vertebrate cell comprising the nucleic acid construct the previous aspect.
In a **fifth aspect,** the invention pertains to a method for recombinant expression of a target protein, comprising a step of culturing under suitable conditions a recombinant vertebrate cell of any one of the preceding claims, wherein the recombinant vertebrate cell further comprises an expression construct encoding the target protein.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a recombinant vertebrate cell, comprising (i) a nucleic acid sequence encoding for a non-vertebrate and/or artificial phosphatidylethanolamine-binding protein (PEBP) and/or (ii) a protein consisting of an amino acid sequence of the non-vertebrate and/or artificial phosphatidylethanolamine-binding protein (PEBP).

As used herein the term "recombinant cell" or "modified cell" refer to a cell that has been manipulated *in vitro,* e.g., using recombinant nucleic acid techniques, to introduce nucleic acid into the cell and/or to modify cellular nucleic acids. Examples of recombinant cells includes prokaryotic or eukaryotic cells carrying exogenous plasmids, expression vectors and the like, and/or cells which include modifications to their cellular nucleic acid (e.g., substitutions, mutations, insertions, deletions, etc., into the cellular genome). An exemplary recombinant cell is one which has been manipulated *in vitro* to express an exogenous protein, such as a phosphatidylethanolamine-binding protein. The term "cell line," as used herein, refers to individual cells, harvested cells, and cultures containing the cells, so long as they are derived from cells of the cell line referred to. A cell line is said to be "continuous," "immortal," or "stable" if the line remains viable over a prolonged time. A "recombinant cell line" in context of the invention shall be understood as a combination of the two definitions provided herein.

The terms "vertebrate" refers to the class of species that comprise all animals with the subphylum vertebrate. The term "non-vertebrate" shall refer to any animal or plant that does not fall under the definition of a "vertebrate". Hence a non-vertebrate can be a plant, a fungus, prokaryote, or an animal invertebrate, such as worms or insects.

In the context of the invention, phosphatidylethanolamine binding protein (PEBP) refers to any protein of the phosphatidylethanolamine-binding protein family. Proteins of this family are known to bind ATP, opioids and phosphatidylethanolamine. These proteins have been identified in numerous tissues in a wide variety of organisms, including bacteria, yeast, nematodes, plants, drosophila and mammals. In all organisms PEBPs appear to regulate important mechanisms that govern cell cycle, proliferation, differentiation and motility. In plants, the PEBP genes have been shown to be involved in various physiological processes, such as seed germination, floral transition, and seasonal growth adaptation. There, the PEBP gene family can be divided into three subfamilies: *MFT, FT, TFL1.* The *MFT* subfamily exists in both basal land plants, and seed plants (gymnosperms and angiosperms), while the *FT* and *TFL1* subfamilies are exclusively found in flowering plants, including early diverging and eudicot lineages. The *FT* and *TFL1* genes are highly conserved in sequence, but their functions may be antagonistic: *FT* mostly promotes flowering, while *TFL1* represses it. In humans, two PEBS are known: PEBP1, and PEBP4.

An artificial PEBP shall be understood to refer to a PEBP whose sequence was altered by human intervention and therefore does not occur in nature in any organism.

In a preferred embodiment of the invention the vertebrate and/or artificial PEBP is a protein that is heterologous to the species of the vertebrate cell, and/or wherein the nucleic acid sequence is for a heterologous expression of the vertebrate and/or artificial PEBP. The term "heterologous protein expression", as used herein, shall refer to the protein expression of a gene, a nucleic acid or a cDNA, which is foreign to the cell in which the expression occurs ("host cell", or "expression system"). Heterologous (meaning 'derived from a different organism') refers to the fact that the transferred protein was initially cloned from or derived from a different cell type or a different species, and coding genetic material (e.g., "cDNA") was obtained which is then transferred to the host cell. The genetic material that is transferred typically must be within a format that encourages the recipient cell to express the cDNA as a protein (i.e., it is part of an expression vector). Methods for transferring foreign genetic material into a recipient cell include transfection and transduction. The choice of recipient vertebrate cell type is often based on an experimental need to examine the protein's function in detail, and the most prevalent recipients, known as heterologous expression systems, are chosen, among others, for (i) ease of transfer DNA, (ii) capability of creating the protein in a pharmaceutically efficacious form, function, (iii) protein yield, and the like. Within the specific scope of the present invention, the term "heterologous" in context of the expression of protein within a cell means that said expressed protein is not a protein naturally expressed by said vertebrate cell originally, or by the organism the respective cell derived from.

The term "recombinant protein expression" largely overlaps with the term "heterologous protein expression". The term "recombinant" alludes to the fact that "new" (coding) genetic material has been introduced into an expression system, e.g., a cell. Such process results in the formation of a recombinant nucleic acid (e.g., a recombinant DNA), and the host is thus called a recombinant host, e.g., a recombinant cell. One idea behind this process is to produce a protein from one organism (e.g., a human protein) in another organism, e.g., in a cell-based protein expression system, like a CHO cell.

In a preferred variant of this embodiment, the non-vertebrate or artificial PEBPs comprise an amino acid sequence having at least 90%, preferably 95%, more preferably 98%, 99% or 100% sequence identity to the sequences of shown in SEQ ID Nos: 1-622 (plant PEBPs), SEQ ID Nos: 820-928 (insect PEBPs), SEQ ID Nos: 929-1127 (prokaryote PEBPs) and SEQ ID Nos: 1128-1270 (fungi PEBPs).

In another preferred embodiment of the invention the non-vertebrate and/or artificial PEBP is a plant or insect PEBP. In the context of the invention, it is particularly preferred to select the origin of the PEBPs that is transferred to vertebrate is a sequence of a PEBP derived from an insect or a plant - such insect PEBPs comprise an amino acid sequences that is shown in any one of SEQ ID Nos: 820-928, or a sequence that is at least 90%, preferably 95%, more preferably 98%, 99% or 100% identical thereto. Preferably, the sequences are PEBP sequences from a drosophila subspecies (SEQ ID NO: 820-827 & 859-887). In yet another preferred variant of this embodiment, the sequences are comprised of at least one of the PEBP sequences of *Drosophila melanogaster* (SEQ ID NO: 820-827). Most preferably, the non-vertebrate PEBP of the invention comprises an amino acid sequence that is at least 90%, preferably 95%, more preferably 98%, 99% or 100% identical to the sequence of CG7054 (SEQ ID NO: 822) or CG10298 (SEQ ID NO: 820) or CG18594 (SEQ ID NO: 821).

In the context of the invention, if a plant PEBP is selected as a non-vertebrate PEBP, such PEBP is selected from one of the amino acid sequences shown in SEQ ID Nos: 1-622. These sequences do not comprise the most C terminal alpha helix of human PEPB1 (SEQ ID NO: 623). In a preferred embodiment of the invention, the non-vertebrate and/or artificial PEBP consists of an amino acid sequence that when aligned with the sequence of SEQ ID NO: 623 (human PEBP1) does not comprise the most C terminal alpha helix. Preferably, these plant PEBP sequences are comprised of amino acid sequences of the FT-like protein family (SEQ ID NO: 1-222). More preferably, these plant PEBP sequences are key activators of flowering, comprising of amino acid sequences of the FT-like protein family with a distinct YAPGW-Motive in their loop region that is responsible for protein interaction (SEQ ID NO: 1-79). In another variant of this preferred embodiment, the plant based PEBPs are comprising the sequences of the TFL1-like proteins (SEQ ID NO: 175, 223-419). In yet another variant of this preferred embodiment, the plant based PEBPs are comprising of MFT-like proteins (SEQ ID NO: 420-517). In a preferred variant of this embodiment, the sequences correspond to PEBPs of a nicotiana subspecies (SEQ ID NO: 1,2, 46,47, 80-82, 182-184, 358-362, 482-484, 577-578). In a preferred variant of this embodiment, the sequences correspond to PEBPs of a nicotiana tabacum (SEQ ID NO: 1,2, 80-82, 361). In a most variant of this preferred embodiment, the sequence corresponds to the sequence of NtFT4 (SEQ ID NO: 1).

In a preferred embodiment of the invention, the non-vertebrate and/or artificial PEBP comprises, and preferably consists of, an amino acid sequence having at least 80%, 90%, 95%, 96%, 97%, 98%, 99% most preferably 100% sequence identity to a sequence shown in SEQ ID NO: 822 (CG7054), SEQ ID NO: 820 (CG10298), SEQ ID NO: 821 (CG18594) or SEQ ID NO: 1 (NtFT4).

In the present application, NtFT4 refers to the protein NtFT4 (Flowering locus T4, Flowering Locus T-like) of the organism *Nicotiana tabacum* (*common tobacco*)*.* It belongs to the phosphatidylethanolamine-binding protein family and is coded by the gene FT4 (Also known as FT2, or LOC107824964). Additional data on this protein can accessed in uniprot as of 24. February 2021 under the file name J9WPJo. The amino acid sequence of the only known variant of the protein is given in SEQ ID NO: 1.

In the present application, CG10298 refers to the protein CG10298 (also known as GH28351p) of the organism *Drosophila melanogaster* (fruit fly). It belongs to the phosphatidylethanolamine-binding protein family and is coded by the gene BcDNA:GH28351. Additional data on this protein can accessed in uniprot as of 24. February 2021 under the file name Q9VIo8. The amino acid sequence of the only known variant of the protein is given in SEQ ID NO: 820.

In the present application, CG18594 refers to the *drosophila melanogaster* (fruit fly) protein CG18594, also known as LP12095P or the *Drosophila melanogaster* version of PEBP1. It belongs to the phosphatidylethanolamine-binding protein family and is coded by the gene Dmel\Pebp1. Additional data on this protein can accessed in uniprot as of 24. February 2021 under the file name Q9VD01. The amino acid sequence of the only known variant of the protein is given in SEQ ID NO: 821.

In the present application, CG7054 refers to the *Drosophila melanogaster* (fruit fly) protein CG7054, also known as GH14779p. It belongs to the phosphatidylethanolamine-binding protein family and is coded by the gene Dmel\CG7054. Additional data on this protein can accessed in uniprot as of 24. February 2021 under the file name Q9VD02. The amino acid sequence of the only known variant of the protein is given in SEQ ID NO: 822.

Generally, a PEBP used in accordance with the invention to generate a cell line with improved characteristics is a functional variant of any of the herein disclosed PEBP proteins. Preferably, if the invention pertains to a protein with a certain degree of sequence variability (variant PEBP) compared to a natural occurring PEBP, it is a preferred embodiment that the variant PEBP is a functional variant of the natural occurring PEBP. The variants and/or fragments are functional variants/fragments in that the variant sequence has similar or identical functional activity (such protein-protein interaction, and thereby for example inhibition of other proteins such as proteases) characteristics to the protein having the non-variant amino acid sequence specified herein (and this is the meaning of the term "functional variant" as used throughout this specification).

A "functional variant" or "functional fragment" of any of the above amino acid sequences, therefore, is any amino acid sequence which remains within the same protein class as the non-variant sequences. Methods of determining whether an enzyme falls within a particular category are well known to the skilled person, who can determine the enzyme category without use of inventive skill. Suitable methods may, for example, be obtained from the International Union of Biochemistry and Molecular Biology. Hence, a functional variant of a naturally occurring PEBP shall in accordance with the present invention retain all biologic function of the naturally occurring PEBP, but to a variant strength. As mentioned elsewhere, the phosphatidylethanolamine-binding protein (PEBP) family, is a small evolutionarily conserved group occurring in all taxa from bacteria to animals and plants (Kardailsky et al., 1999; Kobayashi et al., 1999), and the person of skill in the art may identify homologs and functional variants thereof.

The term "nucleic acid molecule" is meant to include DNA, RNA and mixed DNA-RNA sequences. In addition to the typically found A, T, U, G and C rsidues, a nucleic acid molecule may also include related residues such as, for example, inosine (I).

The term "polynucleotide" or "oligonucleotide" as used herein refers to a polymeric form of nucleotides of any length, either ribonucleotides or deoxyribonucleotides. This term refers only to the primary structure of the molecule. Thus, this term includes double and single stranded DNA, triplex DNA, as well as double and single stranded RNA. It also includes modified, for example, by methylation and/or by capping, and unmodified forms of the polynucleotide.

In a preferred embodiment of the invention, the vertebrate cell is a mammalian cell. Non limiting examples of mammalian cells comprise Chinese hamster ovary cells (CHO), murine myeloma cells (NSo, Sp2/0, C2C12), murine mammary tumor cells (C127), Baby Hamster Kidney Cells (BHK), hamster kidney cells (HaK) rat myeloma cell lines (YB2/0), monkey kidney cells (COS, Vero, CV-1), Murine Sertoli Cells (TM4), buffalo rat liver cells (BRL 3 A), canine kidney cells (MDCK), mouse fibroblast (NIH/3T3), rhesus monkey lung cells (FRhL-2), rat fibroblast cells (RAT-2), mouse bone marrow cells (FDCP-1, 32D cells), mouse lymphatic cells (BA/F3), rat adrenal gland cancer cells (PC12), as well as transformed primate cell lines, hybridomas, normal diploid cells, and cell strains derived from in vitro culture of primary tissue and primary explants. New animal cell lines can be established using methods well known by those skilled in the art (e.g., by transformation, viral infection, and/or selection). Any eukaryotic cell that is capable of expressing a protein of interest may be used in the disclosed cell culture methods. Numerous cell lines are available from commercial sources such as the American Type Culture Collection (ATCC). In one embodiment of the invention, the cell culture, e.g., the large-scale cell culture, employs hybridoma cells. The construction of antibody-producing hybridoma cells is well known in the art.

In another preferred embodiment of the invention, the vertebrate cell is a human cell. Non limiting examples of human cells comprise human embryonic kidney line (HEK293), human fibrosarcoma cells (HT-1080), human cervical carcinoma cells (HeLa), Human cembryonic retinal cells (PER.C6), human liver carcinoma cells (Hep G2, Huh-7), human lung cells (WI-38, MRC-5), human T-lymphocyte (Jurkat), human foreskin (FS-4), human epidermis cells (A431), human colon carcinoma (COLO 205), enteroendocrine cells (L-cells), human histocytic cells (U937), human leukemia cells (HL60), and/or human breast cancer cells (MCF-7). The cell may be derived from a suitable tissue including but not limited to blood, muscle, nerve, brain, heart, lung, liver, pancreas, spleen, thymus, esophagus, stomach, intestine, kidney, testis, ovary, hair, skin, bone, breast, uterus, bladder, spinal cord, or various kinds of body fluids. The cells may be derived from any developmental stage including embryo, fetal and adult stages, as well as developmental origin i.e., ectodermal, mesodermal, and endodermal origin.

The recombinant vertebrate cell of the invention is preferred, wherein the nucleic acid sequence is extra-chromosomal or intra-chromosomal. The term "extra-chromosomal" in context of a genetic element used herein refers to a genetic element other than a chromosome which is introduced into the cell line of the invention such as a virulence plasmid or which is an exogenous genetic element with which the vertebrate cell is transformed and which is transiently or stably integrated into the chromosome or into a genetic element other than a chromosome which is endogenously harboured such as a plasmid. Such an extra-chromosomal genetic element may be a vector like an expression vector, a vector for homologous recombination or other integration into the chromosome or into a genetic element other than a chromosome which is endogenously harboured such as a virulence plasmid, DNA fragments for homologous recombination or other integration into the chromosome or into a genetic element other than a chromosome which is endogenously harboured such as a plasmid. In contrast the term intra-chromosomal may be understood to relate to the introduction of a genetic element into the chromosomal structure of the recipient cell, for example by introducing the non-vertebrate or artificial PEBP gene sequence into an endogenous gene locus of the recipient cell, preferably into an endogenous PEBP gene.

In another preferred embodiment of the invention, the nucleic acid sequence is operatively linked to one or more genetic elements that allow for an inducible or constitutive expression of the vertebrate and/or artificial PEBP in the recombinant vertebrate cell. The term "inducible expression" is used to describe expression that requires interaction of an inducer molecule or the release of a co-repressor molecule and a regulatory protein for expression to take place. The term "constitutive expression" refers to expression which is not usually inducible.

The term "operatively-linked" means a first nucleic acid sequence linked to a second nucleic acid sequence when the first nucleic acid sequence is placed in a functional relationship with the second nucleic acid sequence. For instance, a promoter sequence of the present invention is operably linked to a coding sequence of a heterologous gene if the promoter affects the transcription or expression of the coding sequence.

The term "promoter region" refers to a DNA sequence that functions to control the transcription of one or more nucleic acid sequences, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, calcium or cAMP responsive sites, and any other nucleotide sequences known to act directly or indirectly to regulate transcription from the promoter.

In another preferred embodiment of the invention, the one or more genetic elements comprises a promoter and/or terminator element, such as a CMV promoter and/or bGH terminator.

The term "heterologous DNA" or "heterologous RNA" refers to DNA or RNA that does not occur naturally as part of the genome or DNA or RNA sequence in which it is present, or in which it is found, a cell or location or locations in the genome or DNA or RNA sequence that differs from that which it is found in in nature. Heterologous DNA or RNA is not endogenous to the cell into which it is introduced, but has been obtained from another cell or synthetically or recombinantly produced. Generally, though not necessarily, such DNA encodes RNA and protein not normally produced by the cell in which the DNA is transcribed or expressed. Similarly exogenous RNA encodes protein not normally expressed in the cell in which the exogenous RNA is present. Heterologous DNA or RNA may also be referred to as foreign DNA or RNA. Any DNA or RNA that one of skill in the art would recognize as heterologous or foreign to the cell in which it is expressed is herein encompassed by the term heterologous DNA or heterologous RNA. Examples of heterologous DNA include, but are not limited to, DNA that encodes a protein, polypeptide, reporter nucleic acid sequence, transcriptional or translational regulatory sequences, selectable or traceable marker protein, such as a protein that confers drug resistance, RNA including mRNA and antisense RNA, and ribozymes.

The term "recombinant polynucleotide" as used herein refers to a polynucleotide of genomic, cDNA, semisynthetic or synthetic origin which, by virtue of its origin or manipulation: (1) is not associated with all or a portion of the polynucleotide with which it is associated in nature and/or (2) is linked to a polynucleotide other than that to which it is linked in nature.

The term "operably linked" refers to the linkage of a DNA segment to another DNA segment in such a way as to allow the segments to function in their intended manners. A DNA sequence encoding a gene product is operably linked to a regulatory sequence when it is ligated to the regulatory sequence, such as, for example, promoters, enhancers and/or silencers, in a manner which allows modulation of transcription of the DNA sequence, directly or indirectly. For example, a DNA sequence is operably linked to a promoter when it is ligated to the promoter downstream with respect to the transcription initiation site of the promoter, in the correct reading frame with respect to the transcription initiation site, and allows transcription elongation to proceed through the DNA sequence. An enhancer or silencer is operably linked to a DNA sequence coding for a gene product when it is ligated to the DNA sequence in such a manner as to increase or decrease, respectively, the transcription of the DNA sequence. Enhancers and silencers may be located upstream, downstream or embedded within the coding regions of the DNA sequence. A DNA for a signal sequence is operably linked to DNA coding for a polypeptide if the signal sequence is expressed as a preprotein that participates in the secretion of the polypeptide. Linkage of DNA sequences to regulatory sequences is typically accomplished by ligation at suitable restriction sites or via adapters or linkers inserted in the sequence using restriction endonucleases known to one of skill in the art.

In another preferred embodiment of the invention, the vertebrate cell expressing the vertebrate and/or artificial PEBP compared to the same vertebrate cell not expressing the vertebrate and/or artificial PEBP has a significantly increased vitality, growth and proliferation.

In the **second aspect,** the invention pertains to a method for producing a cell line with improved characteristics selected from cell vitality, cellular protein expression and cell growth, the method comprising the steps of providing a candidate vertebrate cell of a selected vertebrate species, introducing into the vertebrate cell a non-vertebrate and/or artificial phosphatidylethanolamine-binding protein (PEBP) to obtain a cell with improved characteristics, and culturing the cell with improved characteristics to obtain the cell line with improved characteristic.

Introduction of a genetic element for the expression of a PEBP in accordance with the invention usually comprises a step of stably or transiently transfecting or transducing one or more cells with a genetic construct encoding for the PEBP. The term "gene" is used broadly to refer to any segment of nucleic acid associated with a biological function. Thus, genes include coding sequences and/or the regulatory sequences required for their expression. For example, "gene" refers to a nucleic acid fragment that expresses mRNA, functional RNA, or specific protein, including regulatory sequences. "Genes" also include non-expressed DNA segments that, for example, form recognition sequences for other proteins. "Genes" can be obtained from a variety of sources, including cloning from a source of interest or synthesizing from known or predicted sequence information, and may include sequences designed to have desired parameters.

The terms "gene introduction" or "gene delivery" or "gene transfer" refers to methods or systems for reliably inserting foreign DNA into target cells and include transduction, transfection and transformation. Such methods can result in transient or long term (stable) expression of genes. The term "transduction" refers to the delivery of a DNA molecule to a recipient cell either *in vivo* or *in vitro,* via a replication-defective viral vector, such as, e.g., adenoviral vector. The term "transfection" is used to refer to the uptake of foreign DNA by a vertebrate cell. A cell has been "transfected" when exogenous DNA has been introduced across the cell plasma membrane. Transfection can be used to introduce one or more exogenous DNA moieties, such as a plasmid vector and other nucleic acid molecules, into suitable cells. The term refers to both stable and transient uptake of the genetic material. The term "transformation" refers to a process for introducing heterologous DNA into a cell. Transformed cells are understood to encompass not only the end product of a transformation process, but also transgenic progeny thereof.

The term "vector" refers to any genetic element, such as a plasmid, phage, transposon, cosmid, chromosome, virus, virion, etc., which is capable of replication when associated with the proper control elements, such as a helper virus, and which can transfer gene sequences between cells. Thus, the term includes cloning and expression vehicles, as well as replication-defective viral vectors.

The terms "expression vector" and "expression cassette" refer to a nucleic acid molecule capable of directing expression of a particular nucleotide sequence in an appropriate host cell, typically comprising a promoter operatively linked to the nucleotide sequence of interest which is operatively linked to termination signals. It also typically comprises sequences required for proper translation of the nucleotide sequence. The coding region usually encodes a polypeptide(s) of interest but can also encode a functional RNA of interest, for example antisense RNA or a non-translated RNA, in the sense or antisense direction. The expression cassette comprising the nucleotide sequence of interest can be chimeric, meaning that at least one of its components is heterologous with respect to at least one of its other components. The expression cassette can also be one that is naturally occurring but has been obtained in a recombinant form useful for heterologous expression. Typically, however, the expression cassette is heterologous with respect to the host; i.e., the particular DNA sequence of the expression cassette does not occur naturally in the host cell and was introduced into the host cell or an ancestor of the host cell by a transformation event. The expression of the nucleotide sequence in the expression cassette can be under the control of a constitutive promoter or of an inducible promoter that initiates transcription only when the host cell is exposed to some particular external stimulus. In the case of a multicellular organism such as a plant, the promoter can also be specific to a particular tissue, organ, or stage of development.

In a preferred embodiment of this aspect the non-vertebrate and/or artificial PEBP is a heterologous protein to the selected vertebrate species, preferably the selected vertebrate species of the recombinant cell line.

In a preferred embodiment of the invention, the non-vertebrate and/or artificial PEBP is introduced into the candidate vertebrate cell as genetic expression construct comprising a nucleic acid sequence encoding the non-vertebrate and/or artificial PEBP.

In another preferred embodiment of the invention, wherein the candidate vertebrate cell is a mammalian cell.

In another preferred embodiment of the invention, wherein the candidate vertebrate cell is a human cell.

In another preferred embodiment of this aspect, the non-vertebrate and/or artificial PEBP is a plant or insect PEBP.

In yet another preferred embodiment of this aspect, the vertebrate and/or artificial PEBP consists of an amino acid sequence that when aligned with the sequence of SEQ ID NO: 623 (human PEBP1) does not comprise the most C terminal alpha helix.

In a preferred embodiment of the previous embodiment, the nucleic acid sequence is operatively linked to one or more genetic elements that allow for an inducible or constitutive expression of the vertebrate and/or artificial PEBP in the candidate vertebrate cell.

In another preferred embodiment the one or more genetic elements comprises a promoter and/or terminator element, such as a CMV promoter and/or bGH terminator.

In another aspect there is provided an isolated nucleic acid construct suitable for recombinant expression in a vertebrate cell or cell line, comprising a coding element operatively linked with one or more expression elements which is suitable for regulating protein expression in the vertebrate cell, wherein the coding element comprises a nucleic acid sequence encoding a non-vertebrate and/or artificial phosphatidylethanolamine-binding protein (PEBP).

Another aspect pertains to a method for recombinant expression of a target protein, comprising a step of culturing under suitable conditions a recombinant vertebrate cell of any one of the preceding claims, wherein the recombinant vertebrate cell further comprises an expression construct encoding the target protein.

In this further aspect the present invention relates to a method of producing a protein of interest recombinantly in a cell of the invention, comprising: (a) cultivating the inventive cell under conditions conducive for production of the protein; and (b) recovering the protein. Particularly the protein is a heterologous protein.

Optionally, the method may include a first step of transfecting/transducing an expression construct for the target protein / polypeptide into the cell.

The methods used for cultivation and purification of the product of interest may be performed by methods known in the art.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

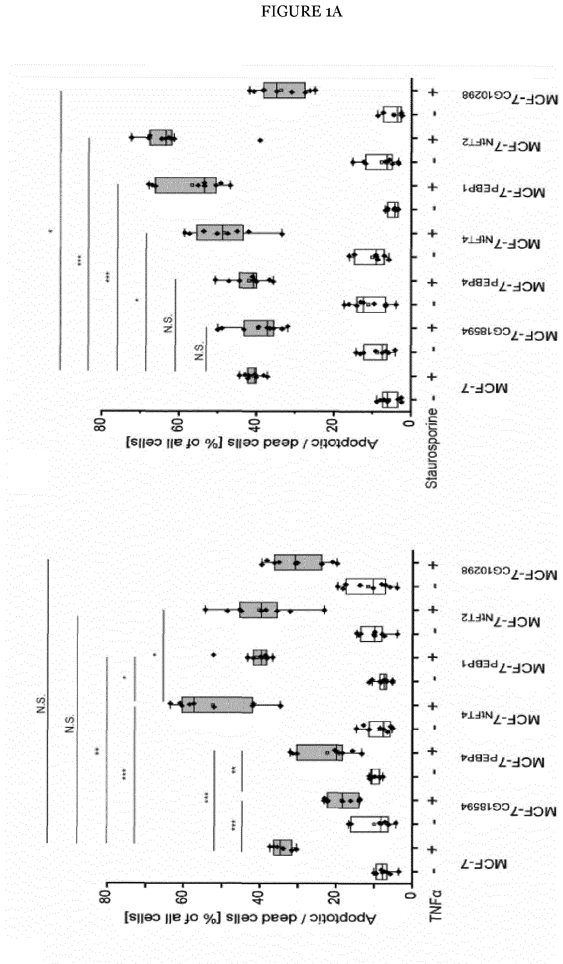

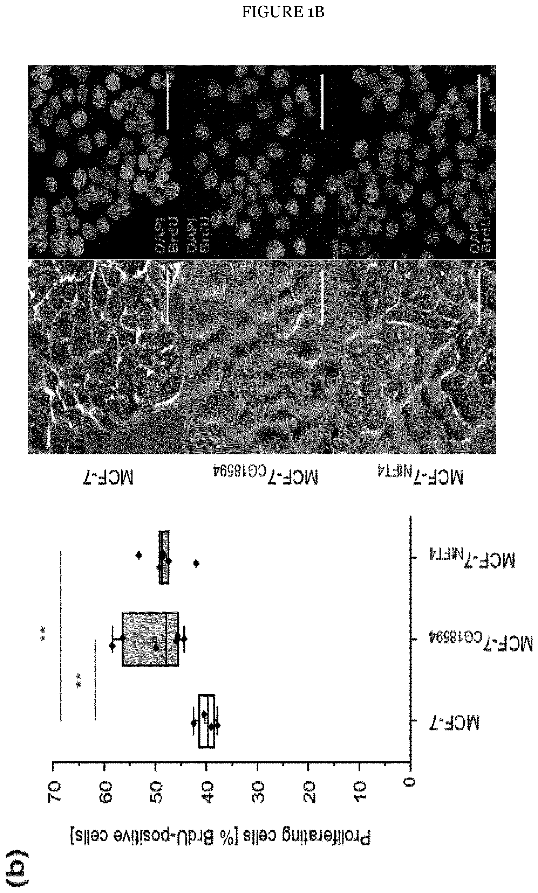

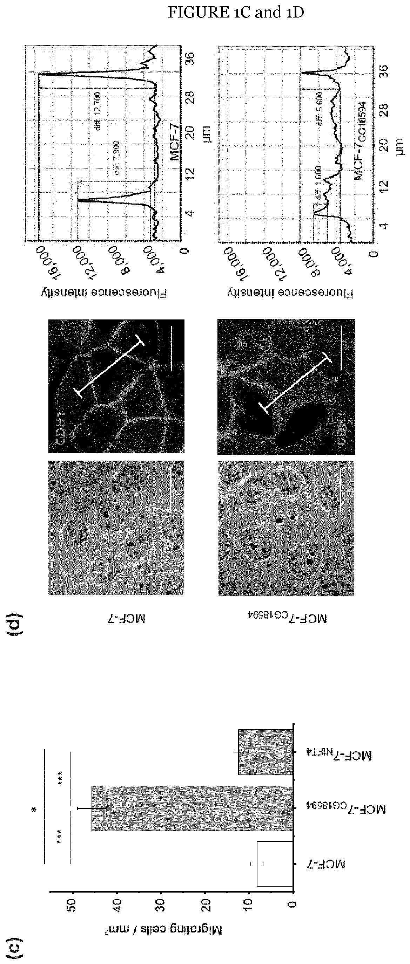

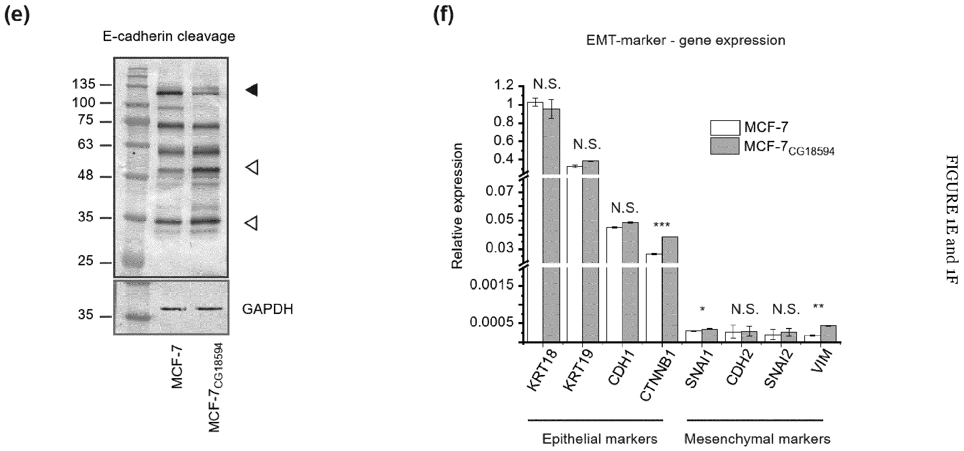

**Figure 1****:** shows *Drosophila* CG18594 PEBP induces mesenchymal traits in MCF-7 cells. Effects of CG18594 on the induction of apoptosis, cell proliferation, migration and the expression and localization of epithelial or mesenchymal markers. **(A)** Apoptosis was induced by 10 ng/mL TNFα (LEFT) or 500 nM staurosporine (RIGHT) for 24 h in MCF-7 cells expressing PEBP1, PEBP4, CG18594, CG10298, NtFT2 or NtFT4, and in MCF-7 control cells. (A) Quantification of apoptotic cells in all cell lines induced by TNFα (LEFT) or staurosporine (RIGHT) (mean ± s.e.m., n=9 biologically independent samples (MCF-7 TNFα-induced n=6), p value from Welch's t-test, *** p < 0.001, ** p < 0.01, * p < 0.05, N.S. not significant). **(B)** Proliferation of MCF-7, MCF-7_{CG18594} and MCF7_{NtFT4} cells. BrdU incorporation by proliferating cells after 2 h was quantified by fluorescence microscopy in relation to total cells stained with DAPI (representative image detail, right) (mean ± s.e.m., n=6 random pictures with > 200 cells, p value from Welch's t-test, ** p < 0.01, scale bar 50 µm). **(C)** Migration of MCF-7, MCF-7_{CG18594} and MCF-7_{NtFT4} cells through the 8-µm pores of a Boyden chamber. Serum served as chemoattractant for 24 h and cells were stained with crystal violet for quantification (mean ± s.e.m., n=10 random pictures, p value from Welch's t-test, *** p < 0.001, * p < 0.05). **(D)** Mesenchymal-like morphological changes of MCF-7C_{G18594} cells. Immunofluorescent staining of E-cadherin in MCF-7 and MCF-7_{CG18594} cells and quantification of fluorescence intensity across a linear ROI (white bar). Peak differences between intensities measured from membrane-bound (high values) and cytosolic E-cadherin (low values) (scale bar = 25 µm). **(E)** Immunodetection of total E-cadherin protein and its cleavage products in MCF-7 and MCF-7_{CG18594} cells. The full-length E-cadherin is indicated by a filled arrowhead and the two main cleavage products that increase in MCF-7_{CG18594} cells (∼50 kDa, ∼33 kDa) are marked by empty arrowheads. GAPDH was used as a reference. **(F)** Gene expression analysis by qRT-PCR of epithelial and mesenchymal markers in MCF-7 and MCF-7_{CG18594} cells in relation to GAPDH (mean ± s.e.m., n=3 biologically independent samples, p value from Welch's t-test, *** p < 0.001, ** p < 0.01, * p < 0.05, N.S. not significant).

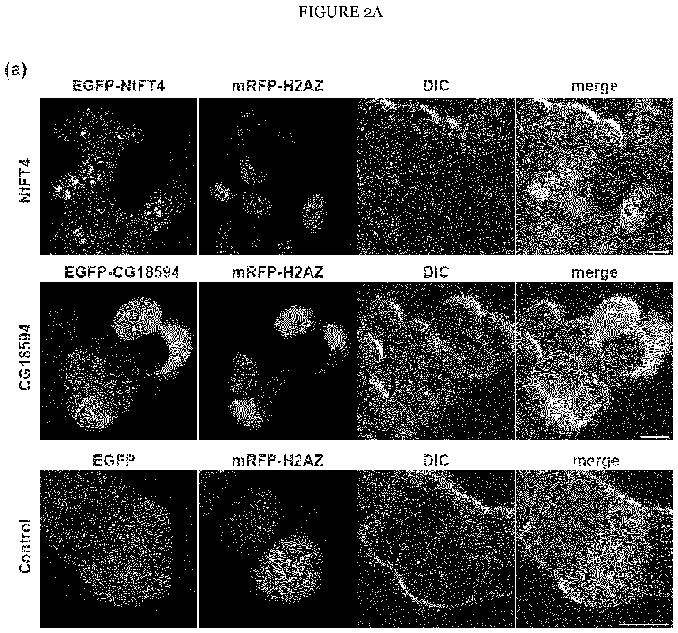

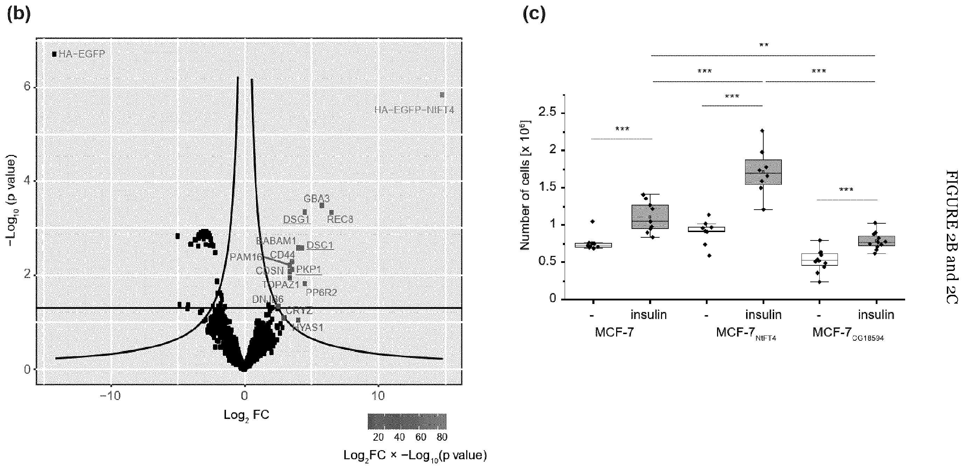

**Figure 2****: NtFT4 forms aggregates, recruits several human interaction partners and thereby amplifies the insulin response in human cells.** (A) Subcellular localization of HA-EGFP-tagged NtFT4 and CG18594 in HEK-293T cells. Cells were transiently transfected with pcDNA3-HA-EGFP (green = HA-EGFP-NtFT4 top line, HA-EGFP-CG18594 middle line and HA-EGFP bottom line) and pcDNA3-myc-mRFP-H2AZ (red) constructs and imaged 24 h post-transfection. scale bar 10 µm (B) Mediators of NtFT4 in human cells based on protein-protein interactions. Interaction partners of HA-EGFP-NtFT4 were identified by protein quantification in eluates. Protein extracts of cells transiently expressing HA-EGFP and HA-EGFP-NtFT4 were precipitated using anti-HA-labeled magnetic beads, digested with trypsin and analyzed by LC-MS/MS. Differential protein abundance was plotted as log2 fold changes in HA-EGFP-NtFT4 samples against corresponding p values. Enriched proteins in the HA-EGFP-NtFT4 samples are indicated by color coding from blue to red. Insulin-responsive adhesion proteins DSG1, DSC1 and PKP1 are underlined (n=3 biologically independent samples, p values were calculated using the Bayes moderated t-test with default settings). (C) Growth response of MCF-7, MCF-7_{NtFT4} and MCF-7_{CG18594} cells to insulin. Serum-starved cells were induced with 40 µg/mL recombinant insulin or with serum-free medium for 48 h and viable cells were quantified (mean ± s.e.m., n=9 biologically independent samples; MCF-7_{NtFT4} insulin-exposed n=8, MCF-7_{CG18594} insulin exposed and starved cells n=12), p value from Welch's t-test, *** p < 0.001, ** p < 0.01).

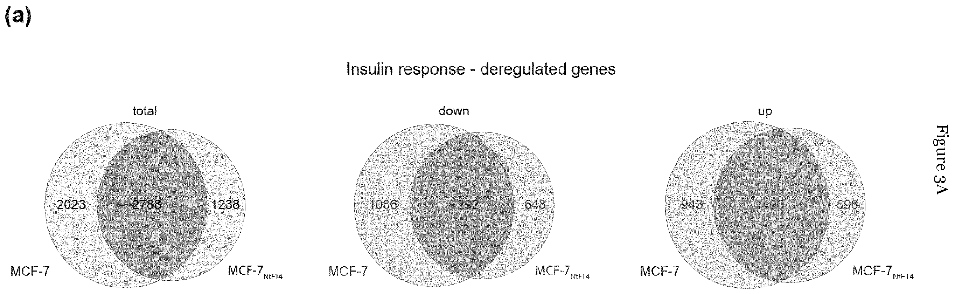

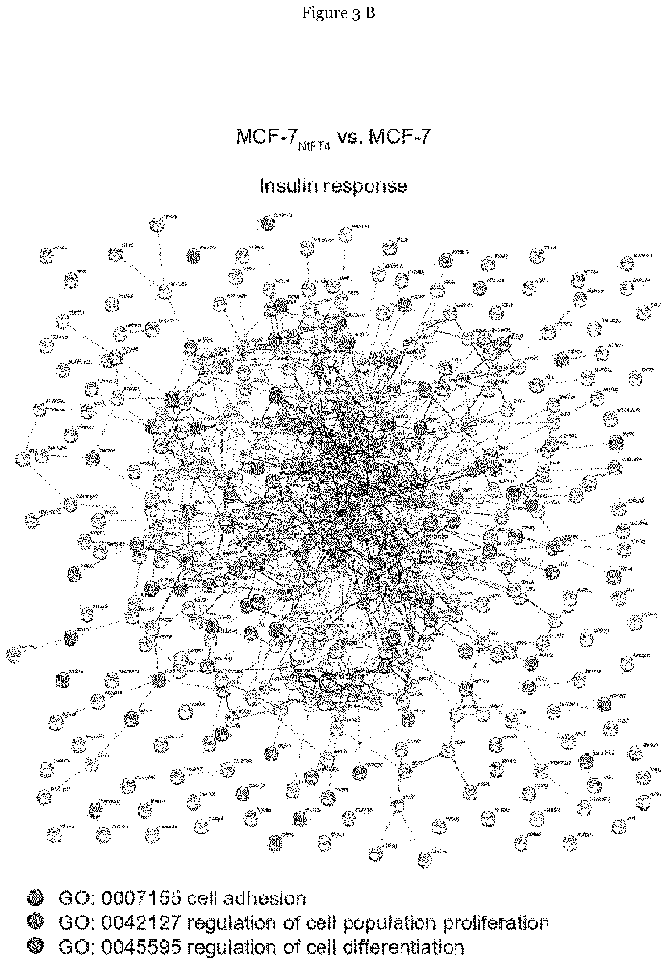

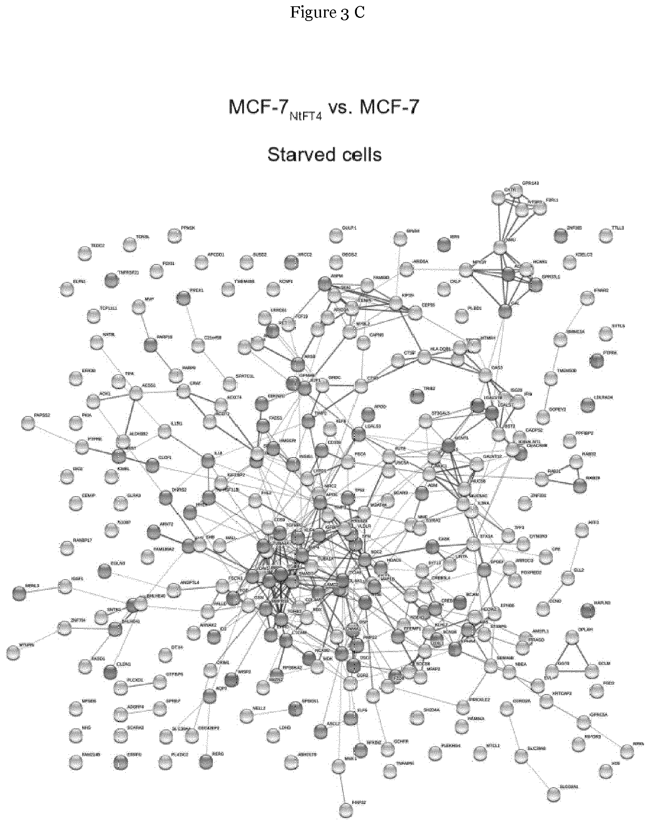

**Figure 3****:** shows **Figure 3****: transcriptome analysis of MCF-7_{NtFT4} cells.** MCF-7 and MCF-7_{NtFT4} cells were exposed to 40 µg/mL recombinant insulin or starved in serum-free medium, followed by whole-transcriptome MACE analysis and pairwise comparisons. **(A)** Overview of deregulated genes in MCF-7 and MCF-7_{NtFT4} cells. Pairwise comparisons between insulin-induced and starved cells. Venn diagrams show common responses in the overlap and differential responses in gene expression (generally deregulated in gray, downregulated genes in blue and upregulated genes in red) to insulin exposure. Genes with an FDR < 0.05 were included (n=3 biologically independent samples). **(B, C)** Interaction network of deregulated genes in MCF-7_{NtFT4} cells in pairwise comparison with MCF-7 cells exposed to insulin (B) and starved in serum-free medium (C). Three enriched biological processes among the deregulated genes are highlighted in blue (cell adhesion), green (regulation of cell population proliferation) or red (regulation of cell differentiation). The query included genes with an FDR < 0.05, log₂FC > 1, and mean normalized reads > 5. Interaction network analysis was carried out using STRING v11.0. FDRs were calculated using the Benjamini-Hochberg procedure.

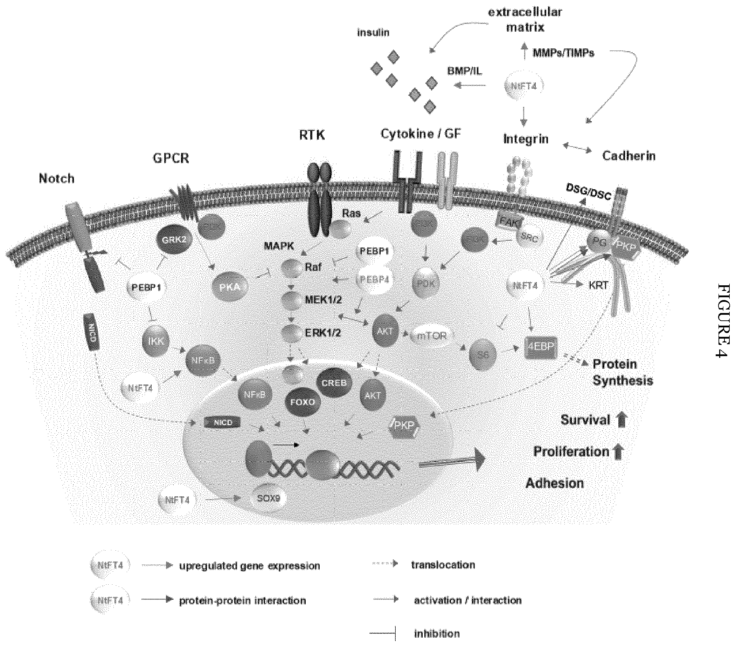

**Figure 4****: Specific PEBP signaling pathway mediated by NtFT4.** Overview of PEBP signaling altered by NtFT4 expression. Human PEBPs regulate cell proliferation and apoptosis by crosstalk among various signaling pathways (RTK-MAPK, GPCR, NFκ(B) Notch and Shh). PEBP1 acts as switch between GPCR and MAPK signaling in a phosphorylation-dependent manner, binding and thereby inhibiting the phosphorylation/activation of either Raf or GRK2. PEBP1 can also bind IKK subunits to inhibit NF-κB signaling and it can inhibit the cleavage and translocation of NCID to the nucleus downstream of Notch1. PEBP4 also intervenes in the MAPK cascade, possibly modulating between MAPK signaling and AKT1. NtFT4 was shown to specifically induce cell proliferation and survival without reducing protein synthesis. A specific function of NtFT4 combines cellular responses that affect cell adhesion, proliferation and differentiation. This involves integrins, desmosomes, insulin-AKT1 and NF-κB signaling. The interaction of HA-EGFP-NtFT4 with desmosomal cadherins 963 and cytosolic PKP1 and JUP suggests a specific role in cell adhesion via desmosomes. The enhanced induction of proliferation following insulin exposure is likely to be mediated by the translocation of desmosomal components to the nucleus where they have been shown to induce proliferation. Additionally, transcriptome analysis revealed enhanced expression of genes associated with cell adhesion and interaction with the extracellular matrix (integrins, collagens and ECM-modifying MMP13, MMP15, MMP17 and TIMP2, TIMP3) linking cell adhesion to the observed increase in the proliferation of MCF-7_{NtFT4} cells. In the cytosol, deregulated genes indicate specific changes in signaling via insulin and Akti by an increase of BMPs (BMP1, BMP4, BMP8B) and IL18. Other key regulators in this pathway are not affected specifically by NtFT4. In the signaling cascade involving the translocation of NF-κB to the nucleus, the expression of Nfkbiz is specifically increased in MCF7-7_{NtFT4} cells. The transcription factor SOX9 is upregulated in the nucleus of MCF7-7_{NtFT4} cells, as well as slight increases in FOXO1 and FOXO3. Insulin exposure did not downregulate 4EBP3 in MCF7-7_{NtFT4} cells (in contrast to MCF-7 cells) and had differing effects on the transcription of various ribosomal S6 kinases. Red arrows indicate transcriptional upregulation. Blue arrows indicate interaction with HA-EGFP-NtFT4. Dashed arrows indicate protein translocation.

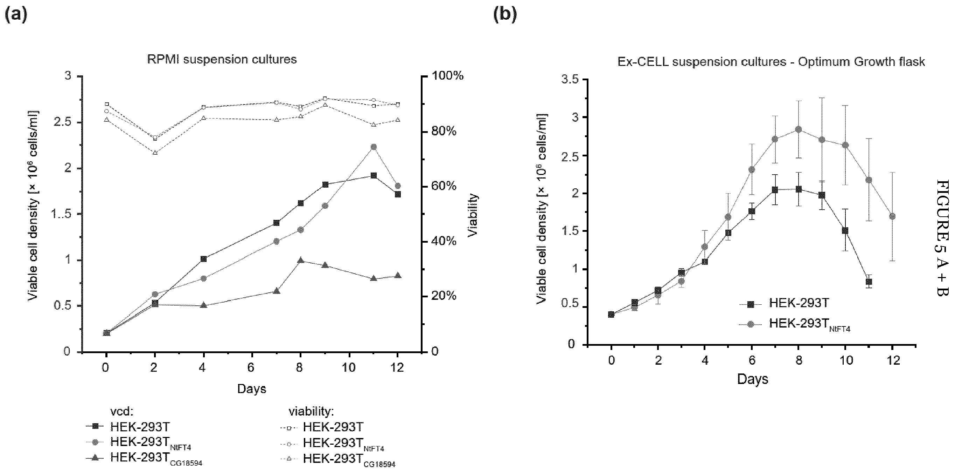

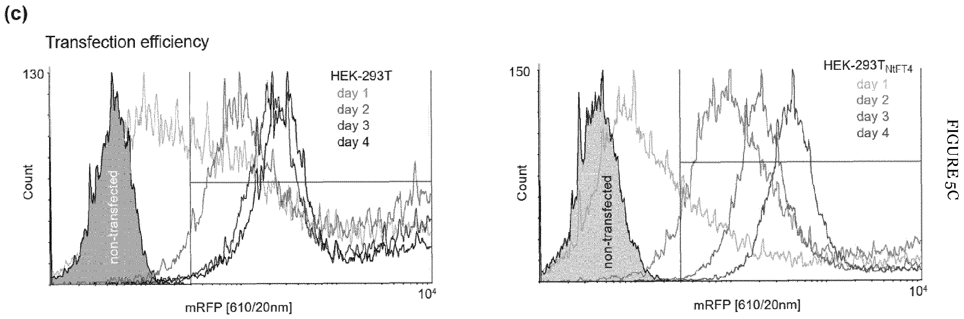

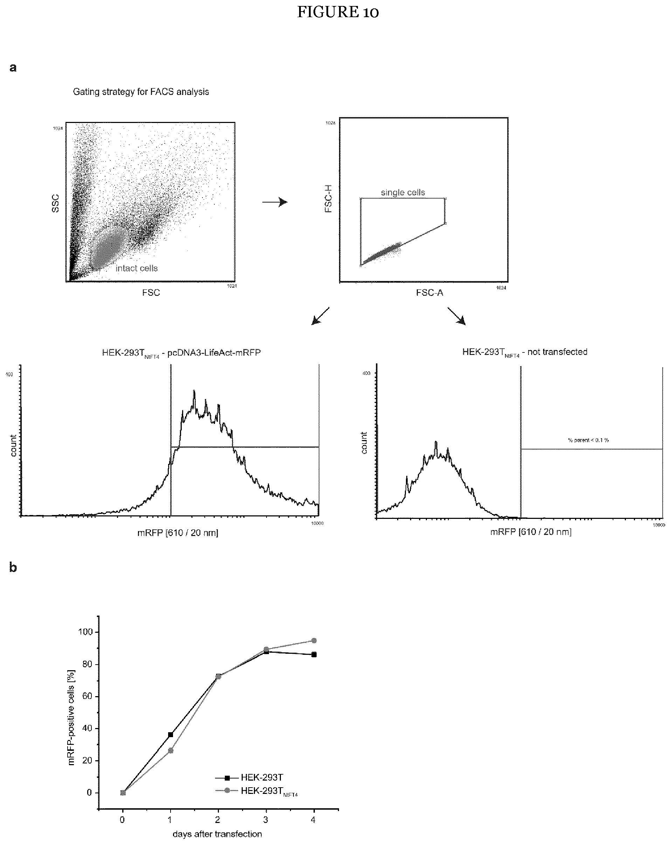

**Figure 5****: Improved growth and transfection efficiency in HEK-293T_{NtFT4} cells. A)** Viable cell density and cell viability of HEK-293T (gray), HEK-293T_{CG18594} (blue) and HEK-293T_{NtFT4} (red) cells adapted to RPMI-1640 + 1% FCS in 40-mL suspension cultures. Viable cell density (solid lines) and cell viability (dashed lines) were measured in batch cultures for 12 days until all lines surpassed their VCD peaks. Representatively shown for n=1 **(B)** Viable cell density and cell viability of HEK-293T (gray) and HEK-293T_{NtFT4} (red) cells grown in 250 mL ExCELL medium in Optimum Growth flasks (Thomson) were measured in batch cultures of three different cell passages of HEK-293T and HEK-293T_{NtFF4} cells (passage ranging between P2-8). n=3 **(C)** Transient transfection efficiency of HEK-293T (left, gray) cells and HEK-293T_{NtFT4} (right, red) cells with pcDNA3Lifeact-mRFP was quantified by FACS analysis. Aliquots of cells were harvested after 1-4 days and after gating for viable, single cells (Figure 10), cells with a distinct mRFP signal were quantified according to the indicated gate (blue). Data were analyzed using Flowing Software v2.5.1.

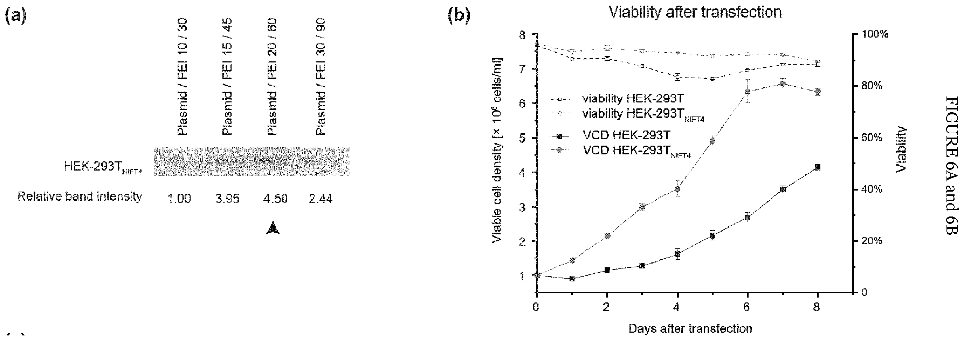

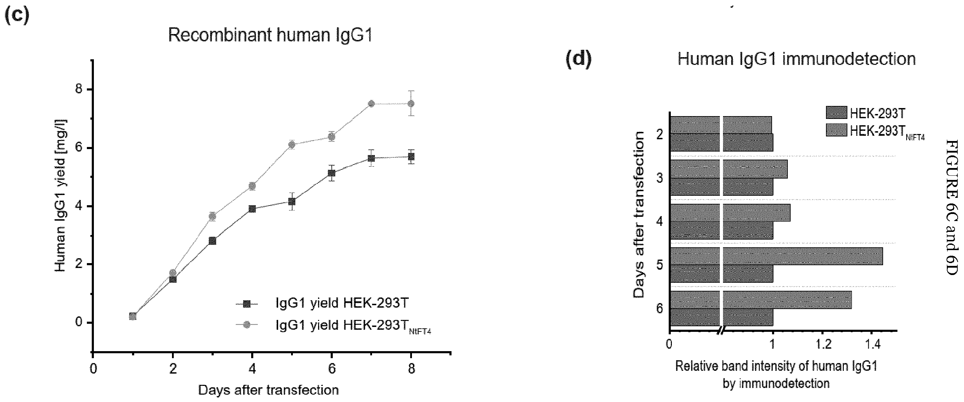

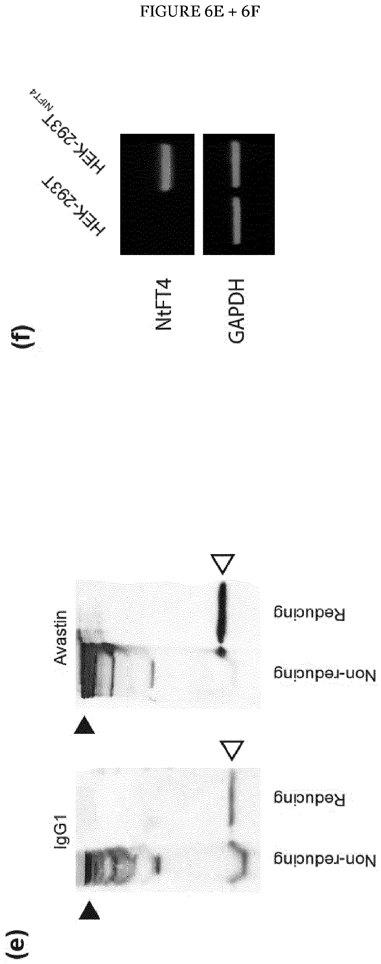

**Figure 6****: Improved protein expression in HEK-293T_{NtFT4} cells.** HEK-293T and HEK-293T_{NtFT4} cells were transiently transfected with pAPT-1E4IgG1 and antibody yield was quantified in the supernatants. **(A)** Immunodetection of IgG1 4 days after transfection with different plasmid and PEI concentrations (plasmid = 10-30 µg/mL, PEI = 30-90 µg/mL). Quantification of band intensities using ImageJ v1.50i revealed highest yield using 20 µg/mL plasmid and 60 µg/mL PEI (arrowhead). **(B)** Viable cell density (solid lines) and cell viability (dashed lines) of HEK-293T (gray) cells and HEK-293T_{NtFT4} (red) cells after transient transfection with pAPT-1E4-IgG1. Aliquots of cells were analyzed on each day after transfection until protein expression reached a steady state (mean ± s.e.m., n=3 biologically independent samples). **(C)** Yield of human IgGi in the supernatants of HEK-293T (gray) cells and HEK-293T_{NtFT4} (red) cells analyzed by ELISA (mean ± s.e.m., n=3 biologically independent samples). **(D)** Quantification of IgGi protein in the supernatants of HEK-293T and HEK-293T_{NtFF4} cells of days 2-6 by western blot using anti-human κ light chain antibody of reduced samples and measuring relative band intensities using ImageJ v1.5oi. **(E)** Immunodetection of IgGi after transient transfection in HEK-293T_{NtFT4} cells and Avastin using non-reducing (left lanes) and reducing (right lanes) loading buffer to analyze correct antibody assembly of heavy and light chains. Under reducing conditions only the kappa-light chains are detected for transiently expressed IgGi (left) and Avastin (right), empty arrowheads. Under non-reducing conditions the assembled antibodies (filled arrowheads) are detected for both the transiently expressed IgGi and Avastin. **(F)** Expression of NtFT4 in HEK-293T_{NtFT4} cells. Prior to experiments, NtFT4 expression was verified by sqRT-PCR using *GAPDH* as the reference gene.

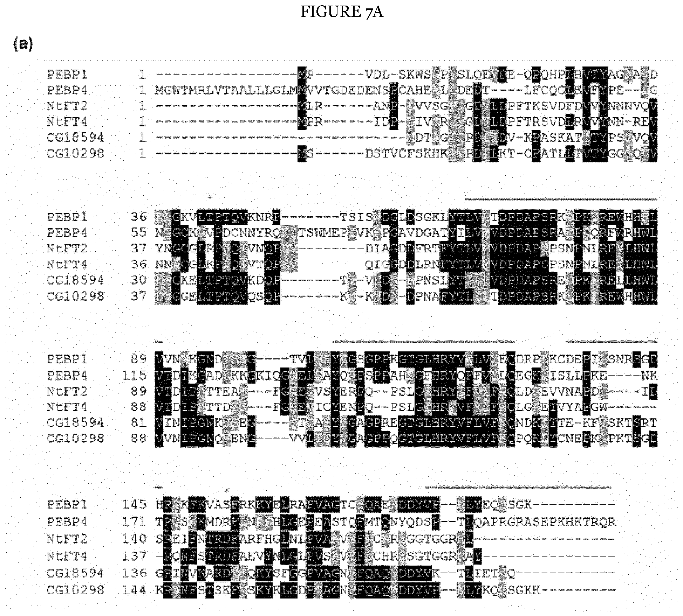

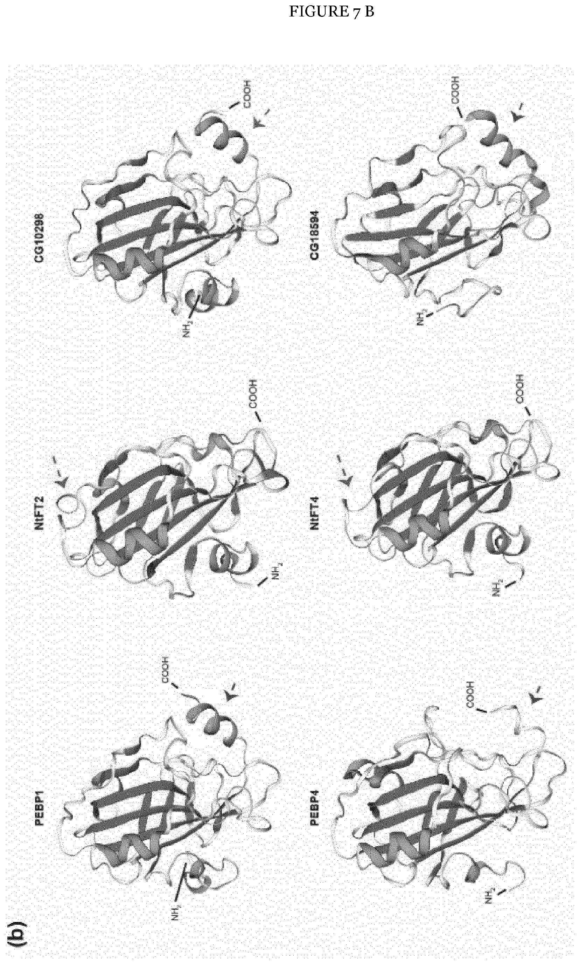

**Figure 7****: Peptide sequence alignment and 3D protein models of PEBP1, PEBP4, NtFT2, NtFT4, CG18594 and CG10298. (A)** Sequences of human PEBP1 (NP_002558.1) and PEBP4 (NP_659399.2), *Nicotiana tabacum* NtFT2 (AFY06688.1) and NtFT4 (AFS17372), and *Drosophila melanogaster* CG18594 (NP_651051.1) and CG10298 (NP_649643.1) were aligned using Clustal Omega (https://www.ebi.ac.uk/Tools/msa/clustalo). Identical amino acids are indicated by black boxes, and similar amino acids by gray boxes. The highly conserved region of PEBPs (binding pocket) is marked by a blue line. Phosphorylation sites in PEBP1 (T42 and S153) are marked by red asterisks and are responsible for the interaction and thus function switches of PEBP1. The decisive motifs of NtFT2 and NtFT4 (NAPDIIDS and YAPGW, respectively) are marked by the green line. In PEBP1, this loop region is affected by phosphorylation of S153. The region of the C-terminal α-helices of animal PEBPs is marked by an orange line. **(B)** Protein models of PEBPs were created using SWISS-MODEL (https://swissmodel.expasy.org/). Human, bovine, mouse and rat RKIP served as the main templates for human and fly PEBPs. These were supplemented with fly PEBP CG7054 and TM16 from *Trichuris muris* for CG10298 and CG18594. Protein models for NtFT2 and NtFT4 were created using FT from *Arabidopsis thaliana* under different conditions as template. The α-helices and β-strands are highlighted in blue and green, respectively. The loop region, which appears to be critical for protein interaction, was turned facing the top right and is flanked by the α-helix containing S153 (PEBP1) and the sequence motifs defining plant NtFT proteins (red arrow). The C-terminal α-helices of animal PEBPs are indicated by gray arrows.

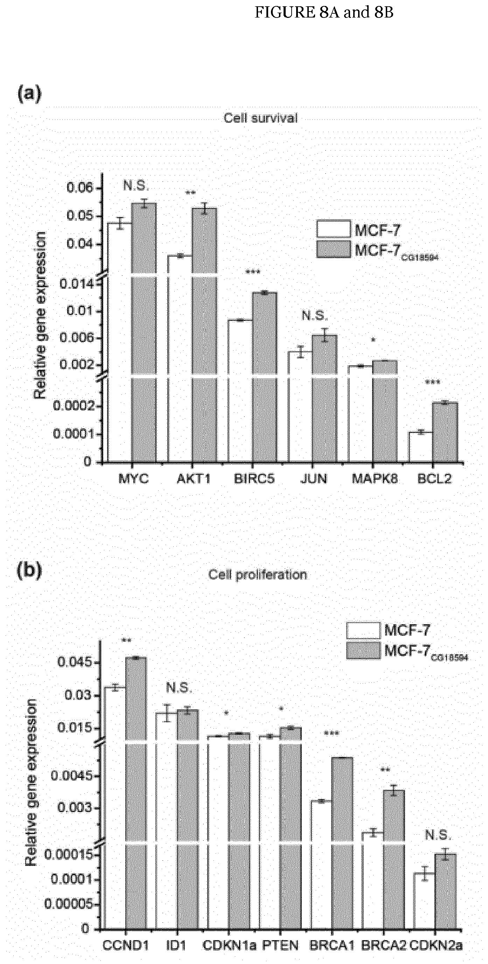

**Figure 8****: Expression analysis of survival and cell cycle related genes.** Analysis of gene expression by qRT-PCR in relation to *GAPDH.* **(A)** Genes related to cell survival or **(B)** cell cycle regulation in MCF-7 and MCF-7_{CG18594} cells (mean ± s.e.m., n=3 biologically independent samples, p value from Welch's t-test, *** p < 0.001, ** p < 0.01, * p < 0.05, N.S. not significant).

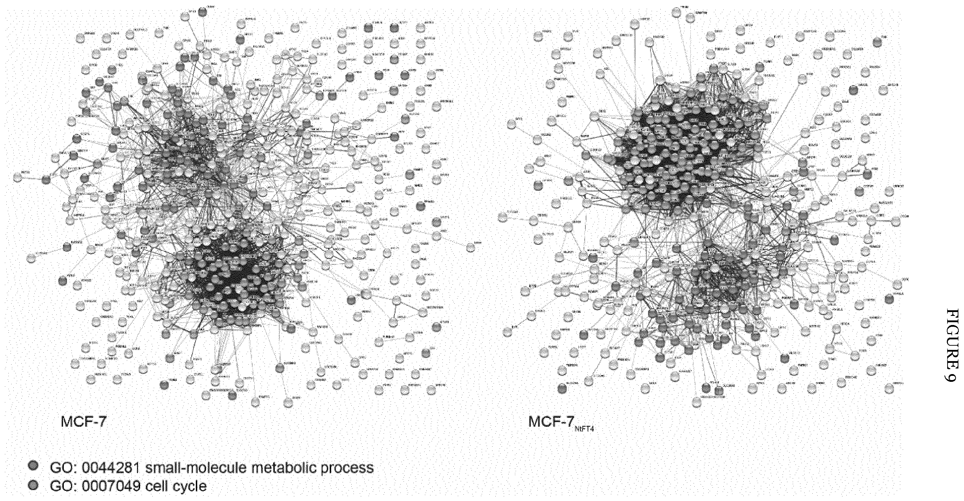

**Figure 9****: Profile plots and interaction network showing differential transcriptome responses to insulin by MCF-7 and MCF-7_{NtFT4} cells.** MCF-7 and MCF-7_{NtFT4} cells were exposed to 40 µg/mL recombinant insulin or starved in serum-free medium, followed by whole-transcriptome MACE analysis and pairwise comparisons. Interaction network of genes upregulated upon insulin exposure in comparison with starved cells in MCF-7 (A) or MCF-7_{NtFT4} cells (B). For MCF-7 cells a total of 478 genes were upregulated and for MCF-7_{NtFT4} cells 386 genes (log2 FC > 1, mean normalized reads > 5, FDR < 0.05). Two gene clusters are highlighted including genes related to small molecule metabolic process (GO: 0044281, blue) and the cell cycle (GO: 0007049, red). FDR values were calculated according to Benjamini-Hochberg.
**Figure 10****: Gating strategy for FACS analysis and quantification of transfection efficiency in HEK-293T and HEK-293T_{NtFT4} cells. (A)** Intact cells were initially gated, excluding cell debris and larger cell aggregates (dot plot top left, red population). For subsequent analysis, only single cells (blue population, top right) were selected, plotting the population of intact cells against the area and height of FSC signals (FSC-A and FSC-H, respectively). For the analysis of transiently transfected cells with pcDNA3-LifeActmRFP, histograms of events detected at 610 ± 20 nm were plotted (bottom). The gate was set using non-transfected control cells with a maximum of 0.1% of the single cells in this gate (green gate, bottom right). The percentage of cells in this gate was quantified using Flowing Software v2.5.1 (bottom left, representative data for HEK-293T_{NtFT4} cells 3 days after transfection) **(B)** Transient transfection efficiency (using 20 µg/mL pcDNA3-LifeAct-mRFP and 60 µg/mL PEI) of HEK-293T (black line) and HEK-293T_{NtFT4} (red line) cells up to 4 days post-transfection.

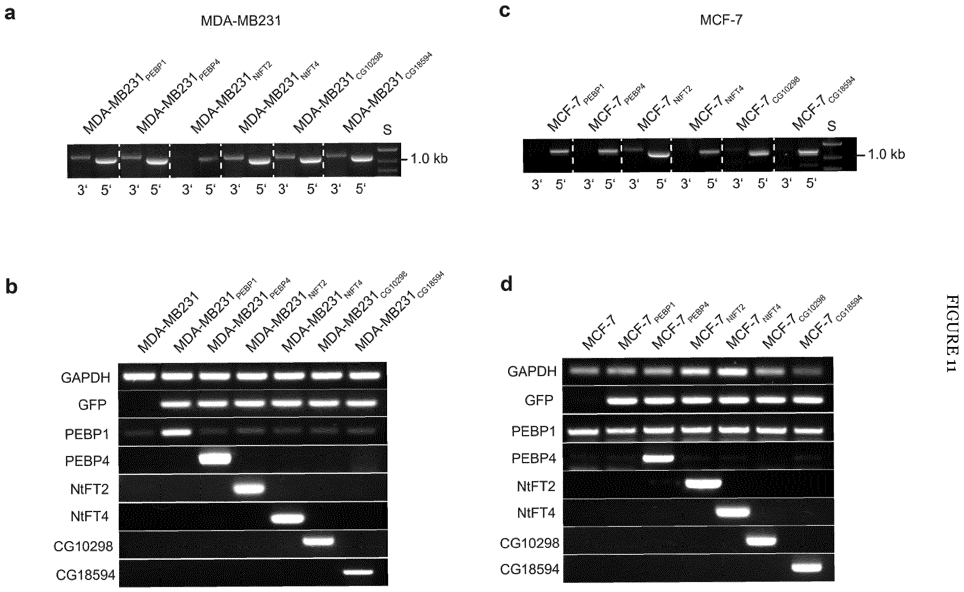

**Figure 11****: Gene integration and expression in MDA-MB231 and MCF-7 cell lines. (A, C)** Verification of correct integration into the *AAVS1* safe harbor locus by 5_{'} and/ or 3' border PCR and agarose gel electrophoresis. A primer pair for each border was used, one primer binding to the adjacent chromosomal region and the other to the integrated sequence. MDAMB231 **(A)** and MCF-7 **(C)** lines carrying the expression cassettes for human PEBP1 and PEBP4, tobacco NtFT2 and NtFT4, and fruit fly CG10298 and CG18594 were verified. **(B, D)** Verification of gene expression in clonal lines expressing the different PEBPs by sqRT-PCR. *GAPDH* expression was included as reference and GFP expression to verify expression of the selection marker GFP-2A-Puromycin. **(B)** Expression of PEBPs in MDA-MB231 and MDAMB231_{PEBP} cells. **(D)** Expression of PEBPs in MCF-7 and MCF-7_{PEBP} cells.

And in the sequences:
The following sequences are of particular exemplary use in context of the invention:

SEQ ID NO: 623 shows sp|P30086|PEBP1_HUMAN Phosphatidylethanolamine-
binding protein 1 OS=Homo sapiens OX=9606 GN=PEBP1 PE=1 SV=3
MPVDLSKWSGPLSLQEVDEQPQHPLHVTYAGAAVDELGKVLTPTQVKNRPTSISWDGLDSGKL
YTLVLTDPDAPSRKDPKYREWHHFLVVNMKGNDISSGTVLSDYVGSGPPKGTGLHRYVWLVY
EQDRPLKCDEPILSNRSGDHRGKFKVASFRKKYELRAPVAGTCYQAEWDDYVPKLYEQLSGK

SEQ ID NO: 624 shows >sp|Q96S96|PEBP4_HUMAN Phosphatidylethanolamine-
binding protein 4 OS=Homo sapiens OX=9606 GN=PEBP4 PE=1 SV=3
MGWTMRLVTAALLLGLMMVVTGDEDENSPCAHEALLDEDTLFCQGLEVFYPELGNIGCKVVP
DCNNYRQKITSWMEPIVKFPGAVDGATYILVMVDPDAPSRAEPRQRFWRHWLVTDIKGADLK
KGKIQGQELSAYQAPSPPAHSGFHRYQFFVYLQEGKVISLLPKENKTRGSWKMDRFLNRFHLG
EPEASTQFMTQNYQDSPTLQAPRERASEPKHKNQAEIAAC

SEQ ID NO: 822 shows >tr|Q9VD02|Q9VD02_DROME GH14779p OS=Drosophila
melanogaster OX=7227 GN=Dmel\CG7054 PE=1 SV=1
MDDIVPDVLDAVPAGTIKVIYGDDLEVKQGNELTPTQVKDQPIVSWSGLEGKSNLLTLLMVDP
DAPTRQDPKYREILHWSVVNIPGSNENPSGGHSLADYVGSGPPKDTGLHRYIFLLYRQENKIEE
TPTISNTTRTGRLNFNARDFAAKHGLGEPIAANYYQAQYDDYVPIRNKTIVG

SEQ ID NO: 820 shows >tr|Q9VI08|Q9VI08_DROME GH28351p OS=Drosophila
melanogaster OX=7227 GN=BcDNA:GH28351 PE=2 SV=1
MSDSTVCFSKHKIVPDILKTCPATLLTVTYGGGQVVDVGGELTPTQVQSQPKVKWDADPNAFYT
LLLTDPDAPSRKEPKFREWHHWLVVNIPGNQVENGVVLTEYVGAGPPQGTGLHRYVFLVFKQ
PQKLTCNEPKIPKTSGDKRANFSTSKFMSKYKLGDPIAGNFFQAQWDDYVPKLYKQLSGKK

SEQ ID NO: 821 shows >tr|Q9VD01|Q9VD01_DROME LP12095p OS=Drosophila
melanogaster OX=7227 GN=Pebp1 PE=1 SV=1
MDTAGIIPDIIDVKPASKATITYPSGVQVELGKELTPTQVKDQPTVVFDAEPNSLYTILLVDPDAP
SREDPKFRELLHWLVINIPGNKVSEGQTIAEYIGAGPREGTGLHRYVFLVFKQNDKITTEKFVSK
TSRTGRINVKARDYIQKYSFGGPVAGNFFQAQYDDYVKTLIETVQ

SEQ ID NO: 82 shows TOBAC Flowering locus T-like protein FT2 (Nicotiana tabacum)
MLRANPLVVSGVIGDVLDPFTKSVDFDVVYNNNVQVYNGCGLRPSQIVNQPRVDIAGDDFRTF
YTLVMVDPDAPTPSNPNLREYLHWLVTDIPATTEATFGNEIVSYERPQPSLGIHRYIFVLFRQLD
REVVNAPDIIDSREIFNTRDFARFHGLNLPVAAVYFNCNREGGTGGRHL

SEQ ID NO: 1 shows >tr|J9WPJ0|J9WPJ0_TOBAC Flowering locus T OS=Nicotiana
tabacum OX=4097 GN=FT4 PE=2 SV=1

MPRIDPLIVGRVVGDVLDPFTRSVDLRVVYNNREVNNACGLKPSQIVTQPRVQIGGDDLRNFYT
LVMVDPDAPSPSNPNLREYLHWLVTDIPATTDTSFGNEVICYENPQPSLGIHRFVFVLFRQLGR
ETVYAPGWRQNFSTRDFAEVYNLGLPVSAVYFNCHRESGTGGRRAY

The sequence protocol comprises the following sequence:
**SEQ ID Nos: 1-622** show plant PEBP amino acid sequences,
**SEQ ID Nos: 623-624** show homo sapiens amino acid sequences,
**SEQ ID Nos: 625-819** show mammalian amino acid sequences,
**SEQ ID Nos: 820-928** show insect PEBPs amino acid sequences,
**SEQ ID Nos: 929-1127** show prokaryote PEBP amino acid sequences,
**SEQ ID Nos: 1128-1270** show fungi PEBP amino acid sequences,
**SEQ ID Nos: 1271-1368** show primer nucleic acid sequences of table 1,
**SEQ ID Nos: 1369-1370** show amino-acid sequences of protein interaction motifs.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:
**Example 1: CG18594 induces mesenchymal traits, promoting apoptotic resistance and proliferation in MCF-7 cells.** Initially, the pro-apoptotic activity of PEBP1 and the anti-apoptotic activity of PEBP4 in the human breast cancer cell line MCF-7 were investigated, confirming their reported properties **(****Figure 1A****).** Among the non-human PEBPs, CG18594 in particular conferred pronounced apoptotic resistance **(****Figure 1A****),** increasing the ability of MCF-7 cells to inhibit responses to TNFα, in a similar manner to PEBP4.

The ability of MCF-7_{CG18594} cells to resist apoptosis was surprising because CG18594 sequence alignments and protein models indicated higher similarity to PEBP1 than PEBP4 (42.6% vs. 27.0%, **Figure** 7). In the control line, the ratio of apoptotic MCF-7 cells increased by 26.1% from 8.0% (± 1.5%) without treatment to 34.1% (± 2.7%) in the presence of TNFα. In the two most resistant lines, TNFα increased the proportion of apoptotic cells by only 8.5% (MCF-7_{CG18954}) and 12.3% (MCF-7_{PEBP4}). In contrast, NtFT4 and NtFT2 acted in a similar manner to PEBP1, increasing TNFα sensitivity (MCF-7NtFT4 +43.7%; MCF-7NtFT2 +30.1%; MCF-7_{PEBP1} +33.0%). Finally, CG10298 showed no significant effect on the induction of apoptosis by TNFα **(****Figure 1****).**

CG18594 also induced the proliferation of MCF-7 cells (+25.5% BrdU incorporation in MCF-7_{CG18594} vs. MCF-7, **Figure 1B**)**.** This was confirmed by analyzing the doubling times of MCF-7 cells in monolayer cultures. In T25 flasks, the doubling time of MCF-7_{CG18594} cells declined by 7.6% compared to MCF-7 controls. A more pronounced decline when cells were visualized by live imaging in 384-well plates was observed. Here, the doubling time of the MCF-7_{CG18594} cells (determined by measuring the confluence) was reduced by 61.4%.

The analysis of MCF-7_{CG18594} cell growth also revealed a partial change toward a mesenchymal-like morphology, which was confirmed by the slight loss of apical-basal polarity during MCF-7_{CG18594} cell migration **(****Figure 1C****).** This reflected the altered distribution of E-cadherin in MCF-7_{CG18594} cells, with less distinct localization to the membrane **(****Figure 1D****).** According to qRT-PCR data, the E-cadherin gene (CDH1) was expressed normally, but the abundance of the full-length protein (135 kDa) declined due to enhanced cleavage, which caused the accumulation of cytoplasmic fragments and thus altered E-cadherin signaling (**Figure 1E, F**). The mesenchymal markers N-cadherin, Snail, Snai2 and vimentin were barely detectable whereas the epithelial markers E-cadherin and β-catenin, as well as the cytokeratins Krt18 and Krt19, remained strongly expressed in MCF-7_{CG18594} cells **(****Figure 1F****)**. Even so, various survival genes and cell cycle regulators were slightly induced in the MCF-7_{CG18594} cells **(****Figure 8****)**. CG18594 thus appears to confer some mesenchymal traits (apoptotic resistance, migratory capacity, partial loss of polarity) but does not induce a full epithelial to mesenchymal transition in MCF-7 cells.

**Example 2: NtFT4 amplifies the insulin response in MCF-7 cells.** NtFT4 and NtFT2 increased the sensitivity of MCF-7 cells toward TNFα in a similar manner to PEBP1. However, PEBP1 had no effect on growth and NtFT2 reduced the growth rate of MCF-7 and MDA-MB231 cells, whereas NtFT4 was the only PEBP to induce proliferation in both MCF-7 (+2.03%) and MDA-MB231 cells (+5.67%). The analysis of BrdU incorporation revealed an even greater increase in the proliferation of MCF-7_{NtFT4} cells (+20.7% vs. MCF-7, **Figure 1B**)**,** and live-cell imaging produced a similar result (-22.7% doubling time,). The two latter techniques are likely to be more accurate because they do not require cell detachment prior to counting, which was influenced by the effect of NtFT4 on cell adhesion. The proliferation of MCF-7_{CG18594} was associated with the partial expression of mesenchymal traits, whereas NtFT4 appeared to interfere directly with epithelial cell junction proteins and their cell signaling pathways. The transient expression of HA-EGFP-NtFT4 together with myc-mRFP-H2AZ revealed the different subcellular localization of NtFT4 and CG18594 **(****Figure 2A****)**. Whereas HA-EGFP-CG18594 was located uniformly throughout the cell, like HA-EGFP, HA-EGFP-NtFT4 accumulated in the nucleus and also appeared to form aggregates in the cytoplasm.

Immunoprecipitation of HA-EGFP-NtFT4 interaction complexes followed by LC-MS/MS revealed that multiple NtFT4 interaction partners were associated with epithelial cell adhesion, especially components of desmosomes. These not only stabilize cell junctions but also act as components in the insulin signal transduction pathway, translocating from the membrane to the nucleus to induce proliferation (see **Figure 2B****,** Fehler! Verweisquelle konnte nicht gefunden werden.). Therefore MCF-7_{NtFT4} and MCF-7_{CG18594} cells were investigated for their response to insulin. Although cell numbers also increased in untreated cultures, insulin exposure triggered the rapid proliferation of MCF-7_{NtFT4} cells (+243.3%) over a period of 48 h compared to MCF-7 (+121.8%) and MCF-7_{CG18594} cells (+57.7%; **Figure 2C**).

. Transcriptome analysis of MCF-7 and MCF-7_{NtFT4} cells, either starved in serum free medium or exposed to insulin, revealed 4811 differentially regulated genes (FDR < 0.05) in MCF-7 cells and 4026 differentially regulated genes in MCF-7_{NtFT4} cells following insulin treatment **(****Figure 3A****).** 2023 genes were identified that were specifically deregulated in MCF-7 cells and 1238 specifically deregulated in MCF-7_{NtFT4} cells, indicating differing responses to insulin induction.

The differential gene expression in MCF-7_{NtFT4} and MCF-7 cells cultivated under starvation conditions or in the presence of insulin was also analyzed. These data support the previous assumption as 3300 (exposure to insulin) and 1794 (starved cells) genes were observed with significant differential expression (FDR < 0.05) between MCF-7_{NtFT4} and MCF-7 cells. But only genes with a normalized read count > 5 and a |log2FC| > 1 were included for further analysis. These genes were analyzed using STRING to identify differentially affected processes. The comparison of MCF-7_{NtFT4} and MCF-7 cells grown in medium containing insulin **(****Figure 3B****)** revealed several significantly enriched processes among the 471 deregulated genes that were included in the query. The GO terms of special interest to narrow down the function of NtFT4 in human cells were *regulation of cell differentiation* (84 genes deregulated in MCF-7_{NtFT4} cells, FDR = 4.98 × 10⁻⁹), *regulation of cell population proliferation* (75 genes deregulated in MCF-7_{NtFT4} cells, FDR = 3.62 × 10⁻⁷) and *cell adhesion* (45 genes deregulated in MCF-7_{NtFT4} cells, FDR = 1.23 × 10⁻⁵). Many of these genes were upregulated in MCF-7_{NtFT4} cells, whereas no enriched processes were identified among the downregulated genes. In starved cells **(****Figure 3C****),** the overall number of deregulated genes was lower (318 genes included in the query), but the GO terms *regulation of cell differentiation* (61 genes deregulated in MCF7_{NtFF4} cells, FDR = 7.89 × 10⁻⁷), *regulation of cell population proliferation* (49 genes deregulated in MCF-7_{NtFT4} cells, FDR = 1.20 × 10⁻⁴) and *cell adhesion* (32 genes deregulated in MCF-7_{NtFT4} cells, FDR = 1.60 × 10⁻⁴) were again significantly enriched and also mainly found among the upregulated genes.

Significant upregulation of several genes encoding members of the integrin (ITGA6, ITGAV, ITGA2 and ITGB1), collagen (COL4A6 and COL5A1) and galectin (LGALS7B, LGALS3 and LGALS1) families revealed that cell-cell and cell-matrix interactions are affected by the expression of NtFT4. Some individual metalloprotease genes were influenced by NtFT4 (MMP13, ADAM29) but there was no major transcriptional reprogramming of the MMP or ADAM families. The comparison of insulin-exposed and starved cells revealed the upregulation of several genes in the *cell cycle* and *small molecule metabolic process* categories in both MCF-7 and MCF-7_{NtFT4} cells **(****Figure 9****)**. In MCF-7_{NtFT4} cells, 108 of the 386 genes upregulated by insulin exposure were related to the cell cycle, compared to 76 of 478 insulin-induced genes in MCF-7 cells. These 108 genes included 48 that were specifically or more strongly upregulated in MCF-7_{NtFT4} cells. Several of these genes are involved in the DNA damage response or cell cycle signaling and regulation, whereas others play a role in the assembly of the kinetochore and centrioles or are directly involved in cytokinesis.

Taken together, these data show that the expression of NtFT4 in human cells accelerated their growth via the specific deregulation of genes linking cell adhesion (or rather cell surface signaling) with intrinsic cues controlling cell proliferation and differentiation **(****Figure 3B****).** The growth-promoting effects of insulin were enhanced in MCF-7_{NtFT4} cells, but proliferation was also induced in the absence of this growth factor by the upregulation of genes involved in intercellular signaling and interactions with the extracellular matrix (*ITGA6, ITGAV, COL5A1, LGALS7, SPOCK1*), signal transduction (*TRIB2, BMP4, CASK*) and transcriptional regulation (*SOX9*) **(****Figure 4****).** These findings suggest two major application routes for NtFT4, namely the development of novel therapies targeting PEBPs and the utilization of plant PEBPs to improve cell growth in human cell lines used for recombinant protein production.

**Example 3: Improved growth of HEK-293T cell suspension cultures expressing NtFT4.** A major drawback of mammalian (particularly human) cells for heterologous protein expression is their susceptibility to loss of vigor under unfavorable conditions, resulting in poor growth. This often occurs during scale-up, because the optimal density or medium composition achieved at lower scales can be disrupted. Cells growing in suspension are also more susceptible to growth suppression due to anoikis and mechanical stress. Therefore, human HEK-293T cells were used, which can be grown as monolayers or suspension cultures, to investigate the effect of NtFT4 and CG18594 on growth and thus determine whether these proteins can improve growth and performance in a biotechnologically relevant human cell line.

HEK-293T cells were established that stably express NtFT4 or CG18594 before converting them to suspension cultures and adapting them to animal component-free Ex-CELL 293 medium. Then the viable cell density (VCD) was analyzed in different culture conditions.

HEK-293T_{CG18594} cells showed impaired growth and viability, whereas the growth of HEK-293T_{NtFT4} cells improved at all stages. Therefore, these cells were selected for further analysis **(****Figure 5****)**. The maximum VCD increased by 13.5% in serum-containing RPMI medium (from 2.15 × 10⁶ to 2.44 × 10⁶ cells/mL, **Figure 5A**) and by 30.1% in the Ex-CELL 293 medium (from 2.59 × 10⁶ to 3.37 × 10⁶ cells/mL; **Figure 5B**). In the latter, the VCDs of three different passages were measured to also take passage number into account. Passage number had an effect on VCDs, as shown by variations in the highest VCDs across the three passages of HEK-293T and HEK-293T_{NtFT4} cells (VCDₘₐₓ HEK-293T: 1.89 × 10⁶ - 2.50 × 10⁶ cells/mL; VCDₘₐₓ HEK-293T_{NtFF4} 2.31 × 10⁶ - 3.81 × 10⁶ cells/mL). However, the HEK-293T_{NtFF4} cells always achieved a higher VCD compared to the parallel HEK-293T cultures (VCDₘₐₓ HEK-293T_{NtFT4} +49.9%, +21.7%, and +52.6%, respectively). This increase in VCD matched the response of MCF-7_{NtFT4} cells to insulin, reflecting the presence of recombinant insulin as a major growth factor in complete ExCELL medium.

In theory, accelerated growth should lead to shorter cultivation times and higher cell densities, which should in turn achieve higher productivity in terms of protein expression. HEK-293T_{NtFT4} cells thus meet the primary criterion for an optimized expression platform. Next, the impact of NtFT4 expression on transfection efficiency was determined by monitoring transfection over 4 days using a non-secreted version of the marker protein mRFP. Multiple transfection parameters were tested (such as different reagents and DNA concentrations, durations and media) but generally observed no differences in transfection efficiency between HEK-293T_{NtFT4} and HEK-293T cells **(****Figure 5C****)**. In each case, the peak of 85% mRFP-positive cells was reached after 3 days based on our threshold gates **(****Figure 10****).** A slightly higher frequency of mRFP-positive HEK-293T cells was observed compared to HEK-293T_{NtFF4} cells on day 1, but no difference or a slightly higher frequency in HEK-293T_{NtFT4} cells thereafter. The HEK-293T_{NtFT4} cells were also found to be more tolerant toward the transfection procedure. Using high-cell densities (20 × 10⁶ cells/mL) during transfection as previously recommended for Ex-CELL medium, it was possible to apply 20 µg/mL of plasmid DNA and 60 µg/mL linear polyethylenimine (PEI) without affecting the viability of HEK-293T_{NtFF4} cells **(Table 1).**

**Table 1: Viability of HEK-293T_{NtFF4} cells after transfection at high cell densities. HEK-293T_{NtFT4} cells were transiently transfected with pcDNA3-Lifeact-mRFP and the impact of different plasmid and PEI concentrations on cell viability and viable cell densities was analyzed for 6 days after transfection. Viability and viable cell density were measured using a Vi-Cell XR cell counter.**

| | **Viability** | | **VCD** | |
|---|---|---|---|---|
| | 10 µg/mL plasmid 30 µg/mL PEI | 20 µg/mL plasmid 60 µg/mL PEI | 10 µg/mL plasmid 30 µg/mL PEI | 20 µg/mL plasmid 60 µg/mL PEI |
| **Day 2** | 94.9% | 95.6% | 1.63 × 10⁶ | 1.45 × 10⁶ |
| **Day 3** | 95.2% | 96.5% | 1.89 × 10⁶ | 1.82 × 10⁶ |
| **Day 6** | 96.4% | 95.4% | 4.22 × 10⁶ | 3.75 × 10⁶ |

The VCD tends to decrease over time in highly-productive transient expression experiments (particularly in batch cultures) but the viability of the HEK-293T_{NtFT4} cells returned to >90% by the second day after transfection and, in contrast to HEK-293T cells, the VCD increased throughout the experiments. Recombinant protein production was quantified by expressing a human IgGi antibody in HEK-293T and HEK-293T_{NtFT4} cells. The expression level was optimized by adapting plasmid and PEI concentrations in HEK-293T_{NtFT4} cells **(****Figure 6A****),** and then compared the two most promising concentrations of plasmid and PEI in both cell lines and used 20 µg/mL plasmid and 60 µg/mL PEI for subsequent comparisons of the two cell lines **(****Figure 6 B-D****).** The VCD of the HEK-293T_{NtFT4} cells again increased rapidly after transfection from 1 × 10⁶ to 6.36 × 10⁶ cells/mL (± 3.25 × 10⁵) after 6 days, whereas that of the HEK-293T cells increased from 1 × 10⁶ to 2.69 × 10⁶ cells/mL (± 1.31 × 10⁵) under the same conditions **(****Figure 6B****).** Importantly, the enhanced proliferation of HEK-293T_{NtFT4} cells did not come at the expense of lower protein yields. By day 2, the concentration of IgGi in the HEK-293T_{NtFT4} cell medium was already 15.67% higher than in the corresponding HEK-293T cultures, and by day 5 the HEK-293T_{NtFT4} cells surpassed the HEK-293T cells by 46.75% **(****Figure 6C, D****).** After 7 days, the secreted IgGi concentration reached a steady state (7.51 ± 0.07 mg/L in the HEK293T_{NtFT4} cultures, compared to 5.64 ± 0.29 mg/L for the HEK-293T cells). By day 8, the IgGi yield of the HEK-293T_{NtFT4} cells (7.52 ± 0.43 mg/L) exceeded the HEK-293T cells (5.70 ± 0.24 mg/L) by 31.95%. The HEK-293T cells never reached the level that HEK293T_{NtFT4} cells had already achieved by day 5 (6.11 ± 0.15 mg/L). Also, the assembly of the heavy and light chains of the secreted IgGi antibody was confirmed by immunodetection of the κ-light chain under non-reducing conditions **(****Figure 6E****).**

The Materials and Methods show:
**Experimental Design:** The functionality of non-mammalian PEBPs was analysed using human breast cancer cell lines because the functions of the human proteins PEBP1 and PEBP4 have previously been characterized in detail using these models. Two PEBPs were selected each from tobacco (*Nicotiana tabacum*) and fruit fly (*Drosophila melanogaster*)*.* The two FT-like proteins NtFT2 and NtFT4 are representative of one subclade of plant PEBPs (the other two subclades found in angiosperms are the TFLi-like and MFT-like proteins), and were selected to see whether their functional specificity would transfer to a non-plant host. Furthermore, two poorly-characterized fruit fly PEBPs were also selected (CG18594 and CG10298) which are similar to human PEBP1. Analysis of cell line growth and exposure to apoptosis-inducing agents led us to focus on NtFT4 and CG18594, the two PEBPs with the strongest and most consistent effects in the human cells. HEK-293T rather than HEK-293 or either of the commercially used HEK-293 derivatives were selected because the former is a major model cell line for biomedical research and is also used as an expression platform for recombinant proteins and viral particles.
**Reagents, plasmids and cloning:** Reagents were purchased from Thermo Fisher Scientific or Sigma-Aldrich if not otherwise stated. Incubations were carried out at room temperature if no temperature is specified. Primers used for cloningTo create stable cell lines expressing different PEBPs, the Genome-TALER human AAVS1 safe harbor gene knock-in kit (GeneCopoeia) was used. For stable transfection of MCF-7, MDA-MB231 and HEK-239T cells with PEBP1, PEBP4, NtFT2, NtFT4, CG18594 and CG10298 constructs, the coding sequces were amplified by PCR using primers with attached restriction sites and were transferred to vector DC-DON-SH1 by restriction and ligation. For the immunoprecipitation of PEBPs, the codon-optimized NtFT4 coding sequence was cloned in frame with HA-EGFP and transferred to vector pcDNA3 by amplifying each segment, digesting the products with restriction enzymes SpeI/XhoI (HA-EGFP), XhoI/ApaI (NtFT4) and SpeI/ApaI (pcDNA3) and ligating them (all restriction enzymes were from New England Biolabs). Vectors pcDNA3-HA-EGFP-CG18594 or pcDNA3-HA-EGFP-NtFT4 and pcDNA3-myc-mFRP-H2AZ were used for colocalization studies, and were prepared by restriction ligation using XhoI/XbaI. The vector pcDNA3-LifeAct-mRFP was used to measure transfection efficiency in the stable cell lines already expressing cytoplasmic GFP. Therefore, the coding sequences for LifeAct (Ibidi) and mRFP were attached by PCR and transferred the product to pcDNA3 using KpnI/XbaI. All plasmids were verified by sequencing. Vector pAPT-1E4-IgG1, encoding a human IgGi with a κ light chain, was a kind gift from Dr. Nicole Raven (Fraunhofer IME, Aachen, Germany).
**Cell Culture:** Adherent MCF-7, MDA-MB231 and HEK-293T cells were grown in RPMI-1640 GlutaMAX medium with 5% fetal calf serum (FCS) and a 1% antibiotic-antimycotic mix in six-well plates for transfection and in T25 and T75 flasks. HEK-293T cells and their derivatives were converted to shaking suspension cultures either in complete RPMI-1640 medium by reducing the serum to 1% in steps or by immediate transfer to Ex-CELL-293 medium with 10 mM HEPES and 6 mM L-glutamine. Suspension cultures were incubated on an orbital shaker at 110 rpm in a 5% CO₂ atmosphere at 37 °C with a relative humidity of ∼93%. The cells were cultivated in 250-mL Erlenmeyer flasks containing 40 mL medium or in 500-mL Optimum Growth flasks (Thomson) containing 250 mL medium.
**Establishing stable cell lines:** Cells were transferred to six-well plates in Opti-MEM for transfections using Lipofectamine 3000 (Thermo Fisher Scientific) according to the manufacturer's protocol. DC-DON plasmids with the expression cassette for different PEBPs were co-transfected with the two TALEN plasmids targeting the AAVS1 safe harbor locus (GeneCopoeia). Successful transfection was verified by fluorescence microscopy to detect expression of the integrated selection marker GFP 2A puromycin. Subsequently, positive cells were selected in complete medium containing 1 µg/mL puromycin dihydrochloride. Stable clonal lines were established by limiting dilution in 96-well plates followed by the selection of colonies grown from single cells. Established MCF-7, MDA-MB231 (Figure 11) and HEK-293T cells expressing different PEBPs were named according to the convention MCF-7_{PEBP}, MDA-MB231_{PEBP} and HEK-293T_{PEBP} (where 'PEBP' refers to the lines collectively or is replaced by the name of a specific protein).
**Verification of single-gene integration and gene expression:** RNA was isolated from each established line using the NucleoSpin RNA kit (MachereyNagel) followed by reverse transcription using PrimeScript RT master mix (Takara) and sqRT-PCR with gene-specific and GAPDH primers. To confirm gene integration, genomic DNA from each cell line was isolated using the NuceloSpin tissue kit for genomic DNA purification (Macherey-Nagel) and specific knock-in was verified by PCR using the primer mixes for the 5' and 3' junction regions (GeneCopoeia).
**Apoptosis assay:** MCF-7_{PEBP} and MDA-MB231_{PEBP} cell lines (PEBP1, PEBP4, NtFT2, NtFT4, CG18594 and CG10298) were seeded at a density of 2.5 × 10⁵ cells/mL in six-well plates. After 24 h the cells were starved for another 24 h by replacing the medium with RPMI plus 1% antibiotic-antimycotic mix but without FCS. Apoptosis was induced by adding 500 nM staurosporine or 10 ng/mL TNFα (100 ng/mL was used for the MDA-MB231 cells) for 24 h. Cells were then harvested with a cell scraper, washed with PBS, filtered through a 35-µm cell strainer (Falcon, Corning) and stained with 7-amino-actinomycin (7-AAD) and Annexin V-BV421 (BD Biosciences) in Annexin V binding buffer (0.01 M HEPES/NaOH (pH 7.4), 0.14 M NaCl, 2.5 M CaCl₂). A FACSCelesta (BD Biosciences) was used for subsequent analysis with the following parameters: 7-AAD excitation = 561 nm, detection = 670/30 nm; Annexin V-BV421 excitation = 405 nm, detection = 450/40 nm. Gating and assignment of populations was carried out as shown in Fig S2. Data were quantified using FACSDiva v8.0.1.1 (BD Biosciences). Populations containing distinguishable late apoptotic and dead cells (7-AAD and Annexin-V positive) were compared.
**Proliferation assay:** Cells were not uniform in their ability to settle after seeding so several methods to analyze the proliferation of adherent MCF-7_{PEBP} and MDA-MB231_{PEBP} cell lines (PEBP1, PEBP4, NtFT2, NtFT4, CG18594, CG10298) have been used. First, cells were counted for 4 days after seeding 3 × 10⁵ cells in three replicates for each time point into T25 flasks in complete medium. At each time point, the cells were harvested, carefully resuspended in 1 mL complete medium and counted using a Vi-Cell XR cell counter (Beckmann Coulter). Using the initial seeding density and the density after 4 days, average doubling times for each replicate were calculated. Cells were also seeded at 500 or 1000 cells per well into 384-well plates (16 wells per cell line, three replicates) and confluency was monitored every 2 h for 48 h using the IncuCyte ZOOM system (Sartorius) allowing doubling times to be calculated. Finally, cell proliferation was measured incorporating 5-bromo-2'deoxyuridine (BrdU). 20,000 cells were seeded into eight-well chamber slides in complete RPMI-1640 medium, added 50 µL 0.3 mg/mL BrdU after 24 h and incubated for 2 h. The cells were then fixed with ice-cold ethanol (70%), treated with 1.5 M HCl and blocked with PBS containing 5% (v/v) FBS with intermediate washing steps in PBS. The cells were incubated with anti-BrdU-Alexa Fluor 488 at 4 °C overnight. For microscopy, cells were washed with PBS + 5% (v/v) FBS, nuclei were counterstained with 4',6-diamidino2-phenylindole (DAPI) and the cells were mounted with anti-fade. BrdU-labeled cells were counted under a DMi8 fluorescence microscope (Leica Microsystems) at six random positions containing at least 300 cells to ensure comparable confluency. To analyze the growth response to insulin, 5 × 10⁵ MCF-7_{PEBP} cells (NtFT4 and CG18594) were seeded in complete medium in T-25 flasks. After settling, the cells were starved for 24 h in serum-free medium before adding 40 µg/mL recombinant human insulin. After 48 h the cells were harvested and counted using a Vi-CELL XR cell counter.
**Migration assay:** The MCF-7_{PEBP} cell lines (NtFT4 and CG18594) were seeded (1 × 10⁵ cells in 100 µL serum-free medium) in 24-transwell Boyden chambers with a pore size of 8 µm (Corning) and complete medium in the lower chamber, and were incubated for 24 h. Medium was then removed from the upper chamber and the non-migrated cells were carefully removed using a cotton swab. The inlet was transferred to a new 24-well chamber containing 4% (w/v) formaldehyde for 30 min at 4 °C. After drying, fixed cells were stained in a 0.1% (w/v) crystal violet for 5 min and washed with water. Random images of migrated cells were captured using a MZ 16 F fluorescence stereomicroscope (Leica Microsystems) and counted using ImageJ v1.50i.
**Immunofluorescence:** MCF-7 and MCF-7_{CG18594} cells were fixed with 4% formaldehyde for 30 min at 4 °C, washed three times with PBS and blocked for 60 min with blocking solution (PBS, 5% FBS, 0.3% Triton X-100) before incubating overnight at 4°C with Anti-E-cadherin (24E10) (Cell Signaling; #3195) in PBS containing 1% BSA and 0.3% Triton X-100. Cells were then washed three times with PBS and incubated with anti-rabbit IgG-Alexa Fluor 546 for 2 h in the dark. Cells were analyzed by fluorescence microscopy using a DMi8 inverted microscope (Leica Microsystems) and fluorescence intensities in linear regions of interest were measured using LAS X software (Leica Microsystems).
**Live cell imaging for** subcellular **localization:** Localization studies using the pcDNA3 vectors containing constructs HA-EGFP, HA-EGFP-NtFT4, HA-EGFP-CG18594 and myc-mRFP-H2AZ were carried out by co-transfecting HEK-293T cells with EGFP plasmids and pcDNA₃-myc-mRFP-H2AZ using Lipofectamine 3000. Cells were transiently transfected in six-well plates in Opti-MEM medium and fluorescence was imaged in living cells 24 h post-transfection using a TCS SP₅ X laser scanning microscope (Leica Microsystems).
**Protein extraction, analysis and western blotting:** Adherent cells were harvested with a cell scraper, washed with cold PBS, and proteins were extracted using 300 µL cold RIPA lysis buffer (25 mM Tris-HCl pH 7.6, 150 mM NaCl, 1% NP-40, 1% sodium deoxycholate, 0.1% SDS containing protease and phosphatase inhibitor cocktails) for 30 min on ice with occasional shaking. Protein concentrations in the extract supernatant were measured using the Pierce Coomassie Plus Protein Assay (Thermo Fisher Scientific) according to the manufacturer's specifications. Proteins were separated by SDS-PAGE and stained using the PAGE Blue protein staining kit or transferred to a 0.2-µm nitrocellulose membrane using the wet Mini Trans-Blot Cell system (Bio-Rad Laboratories). Western blots were probed with the following antibodies: E-cadherin mAb rabbit 24E10 (Cell Signaling, #3195); GAPDH D16H11 rabbit mAb (Cell Signaling, #5174); IGF-I receptor β D23H3 rabbit mAb (Cell Signaling, #9750); phospho-Akt (Ser473, D9E) rabbit mAb (Cell Signaling, #4060); phospho-mTOR (Ser2448, D9C2) rabbit mAb (Cell Signaling, #5536); HA tag rabbit pAb (MBL, #561); human IgG (H+L) alkaline phosphatase (AP) (Thermo Fisher Scientific, #62-8422). Primary antibodies were detected using either anti-rabbit IgG sAb AP and SigmaFast BCIP/NBT tablets or anti-rabbit IgG sAb horseradish peroxidase and the SuperSignal West dura kit, and the signals were detected using a G:Box Chemi (Syngene). Brightness and contrast were optimized using Adobe Photoshop CS6 V13.0.1×64 (Adobe Systems).
**Quantitative PCR:** Gene expression was analyzed by quantitative real-time PCR using Kapa SYBR Fast qPCR Master Mix and the CFX96 Real-Time System (Bio-Rad). Each reaction was carried out in technical triplicates and the primer sequences are provided in **Table 2** Specificity was ensured by melt curve analysis and the sequencing of PCR products, and by including no-template and no-reverse-transcription controls. Individual PCR efficiency was determined using LinReg PCR V2017.0 and relative gene expression levels were normalized to GAPDH following the initial testing of GAPDH, ACTB, GUSB and MRPL32.

**Table 2: Oligonucleotide Sequences used in context of the invention**

| **Name** | **Sequence 5' to 3'** | **Purpose** |
|---|---|---|
| PEBP1 for XbaI | aaatctagaATGCCGGTGGACCTCAGC | cloning of donor plasmids into DC-DON-SH1 backbone |
| PEBP1 rev AscI | agaggcgcgccCTACTTCCCAGACAGCTGCTCG | |
| PEBP4 for XbaI | aaatctagaATGGGTTGGACAATGAGGC | |
| PEBP4 rev AscI | agaggcgcgccCTAGCAGGCAGCTATCTCCG | |
| NtFT2 for XbaI | aaatctagaATGTTAAGAGCAAATCCTTTAG | |
| NtFT2 rev AscI | agaggcgcgccTTATAGGTGACGGCCACC | |
| NtFT4 for XbaI | aaatctagaATGCCAAGAATAGATCCTTTG | |
| NtFT4 rev AscI | agaggcgcgccTTAATATGCGCGGCGG | |
| CG18594 for XbaI | aaatctagaATGGACACCGCCGGCATTAT | |
| CG18594 rev AscI | agaggcgcgccTTACTGGACCGTCTCGATGAGG | |
| CG10298 for XbaI | aaatctagaATGTCCGATTCCACCGTG | |
| CG10298 rev AscI | agaggcgcgccCTACTTCTTGCCAGATAGTTGC | |
| HA-EGFP for SpeI | aaaactagtatgtacccctacgatgtccctgactacgctATGGTGAGCAA GGGC | cloning of pcDNA3-HA-EGFP |
| EGFP rev XbaI | aaaatctagaTTACTTGTACAGCTCGTCCATG | |
| EGFP- rev XhoI | aaaatctagaCTTGTACAGCTCGTCCATG | cloning of pcDNA3-HA-EGFP-NtFT4 |
| NtFT4 for XhoI | aaactcgagATGCCAAGAATAGATCCTTTG | |
| NtFT4 rev ApaI | aaagggcccTTAATATGCGCGGCGG | |
| LifeAct-mRFP for KpnI | acaggtaccatgggcgtggccgacctgatcaagaagttcgagagcatcagc aaggaagagggcgacccaccggtcgccaccATGGCCTCCTCCGAG | |
| mRFP rev XbaI | aaatctagaTTAGGCGCCGGTGGA | |
| DC-DON-SH1 seq for | CGGTGGGAGGTCTATATAAGCAG | sequencing |
| DC-DON-SH1 seq rev | GACAGTGGGAGTGGCACCTTC | |
| CMV for | CGCAAATGGGCGGTAGGCGTG | |
| T7 for | TAATACGACTCACTATAGGGAGA | |
| bGH rev | TAGAAGGCACAGTCGAGG | |
| 5' Junction PCR Primer for | CCGGAACTCTGCCCTCTAAC | verification of genomic integration |
| 5' Junction PCR Primer rev | CCCGTGAGTCAAACCGCTAT | into the aavs1 safe harbor locus |
| 3' Junction PCR Primer for | AGCTATCTGGTCTCCCTTCC | |
| 3' Junction PCR Primer rev | TCCTGGGATACCCCGAAGAG | |
| GFP for | ATCGAGAAGTACGAGGACG | transgene expression - reference gene |
| GFP rev | CACCACGAAGCTGTAGTAGC | |
| PEBP1 for | AGACCCACCAGCATTTCGTG | transgene expression - PEBP expression |
| PEBP1 rev | GGTGGTCTCCAGATCGGTTG | |
| PEBP4 for | TCCTGGATGGAGCCGATAGT | |
| PEBP4 rev | AACTGGTAGCGATGGAAGCC | |
| NtFT2 for | AGATATCCCTGCAACCACAGAAGCAAC | |
| NtFT2 rev | AAACAGCGGCAACAGGCAAATTGAGAC | |
| NtFT4 for | GATATCCCAGCAACTACAGATACAAG | |
| NtFT4 rev | GAAACGGGCAAACCAAGATTGTAAAC | |
| CG18594 for | CCACCATCACCTATCCTTCC | |
| CG18594 rev | GAACTTCTCGGTGGTGATCT | |
| CG10298 for | AGCCAGCCAAAGGTGAAA | |
| CG10198 rev | GGACCTGCACCCACATATT | |
| GAPDH for | CTCTGCTCCTCCTGTTCGAC | gene expression - reference gene |
| GAPDH rev | TTCCCGTTCTCAGCCTTGAC | |
| β-ACTIN for | CACAGAGCCTCGCCTTTGC | |
| β-ACTIN rev | AATCCTTCTGACCCATGCCC | |
| RpL32 for | GGTTACGACCCATCAGCCC | |
| RpL32 rev | TCAATGCCTCTGGGTTTCCG | |
| GUSB for | GCTCCGAATCACTATCGCCA | |
| GUSB rev | CGCACTTCCAACTTGAACAGG | |
| CDH1 for | TTCTGCTGATCCTGTCTGAT | gene expression - epithelial markers |
| CDH1 rev | AGGTGGTCACTTGGTCTTTA | |
| CTNNB1 for | AAGGAGCTAAAATGGCAGTG | |
| CTNNB1 rev | TCCTAAAGCTTGCATTCCAC | |
| KRT18 for | TGACACCAATATCACACGAC | |
| KRT18 rev | TCAATCTGCTGAGACCAGTA | |
| KRT19 for | CACTACTACACGACCATCCA | |
| KRT19 rev | CTCAGCGTACTGATTTCCTC | |
| CDH2 for | ATGTGCCGGATAGCGG | gene expression - mesenchym al markers |
| CDH2 rev | GGCTCACTGCTCTCATATTG | |
| SNAI1 rev | CTTGACATCTGAGTGGGTCTG | |
| SNAI1 for | CTCGAAAGGCCTTCAACTG | |
| SNAI2 for | ATTCGGACCCACACATTAC | |
| SNAI2 rev | CAGAATGGGTCTGCAGAT | |
| VIM for | AGGAGGAGATGCTTCAGAGA | |
| VIM rev | AGCTCCTGGATTTCCTCTTC | |
| AKT1 for | GACCATGAACGAGTTTGAGT | gene expression - cell |
| AKT1 rev | CGTACTCCATGACAAAGCAG | |
| BCL2 for | GAACTGGGGGAGGATTGTG | survival |
| BCL2 rev | AGAAATCAAACAGAGGCCG | |
| BIRC5 for | CGACCCCATAGAGGAACATA | |
| BIRC5 rev | CTAAGACATTGCTAAGGGGC | |
| JUN for | ACTGCAAAGATGGAAACGAC | |
| JUN rev | TGCTCATCTGTCACGTTCT | |
| MAPK8 for | GAGCTCATGGATGCAAATCT | |
| MAPK8 rev | AGGCGTCATCATAAAACTCG | |
| MYC for | GAGACAGATCAGCAACAACC | |
| MYC rev | TTTCAACTGTTCTCGTCGTT | |
| BRCA1 for | CCAGAACAAAGCACATCAGA | gene expression - proliferatio n |
| BRCA1 rev | ATGTTTCCGTCAAATCGTGT | |
| BRCA2 for | TACATGAACAAATGGGCAGG | |
| BRCA2 rev | ATTTCTGCCTTTTGGCTAGG | |
| CCND1 for | TCGGTGTCCTACTTCAAATG | |
| CCND1 rev | ATGGAGTTGTCGGTGTAGAT | |
| CDKN1Afor | CACTGTCTTGTACCCTTGTGC | |
| CDKN1A rev | AAAATGCCCAGCACTCTTAG | |
| CDKN2A for | GAGGGCTTCCTGGACAC | |
| CDKN2A rev | TCAATCGGGGATGTCTGAG | |
| ID1 for | GAATCATGAAAGTCGCCAGT | |
| ID1 rev | TGAAGGTCCCTGATGTAGTC | |
| PTEN for | CAAGATATACAATCTTTGTGCTGA | |
| PTEN rev | TGGTCCTTACTTCCCCATAG | |

**Immunoprecipitation:** HEK-293T cells were seeded in six-well plates and transfected with pcDNA3-HA-EGFP-NtFT4 and pcDNA3-HA-EGFP using Lipofectamine 3000. Immunoprecipitation was carried out using the Pierce Magnetic IP/Co-IP kit (Thermo Fisher Scientific) according to the manufacturer's specifications. Success was confirmed by silver staining of the eluate and input proteins using the Pierce Silver Stain for Mass Spectrometry (Thermo Fisher Scientific) according to the manufacturer's specifications, and by western blot.
**LC-MS analysis:** Immunoprecipitated proteins were reduced in 5 mM DTT for 30 min and alkylated with 14 mM chloracetamide for 30 min. Samples were digested with trypsin at 37 °C overnight. The digest was quenched with 1% formic acid and the peptides desalted with C18 stage tips prior to MS analysis. Dried peptides were redissolved in 2% acetonitrile supplemented with 0.1% trifluoroacetic acid (TFA) for analysis. Samples (0.5 µg) were analyzed using an EASY-nLC 1200 coupled to a Q Exactive HF mass spectrometer (Thermo Fisher Scientific). Peptides were separated on 20-cm frit-less silica emitters (New Objective, 0.75 µm inner diameter) packed in-house with reversed-phase ReproSil-Pur C18 AQ 1.9 µm resin (Dr. Maisch). The column was kept at 50 °C in a column oven throughout the run. Peptides were eluted for 115 min using a segmented linear gradient of 0-98% solvent B (solvent A = 0.5% formic acid in water; solvent B = 80% acetonitrile, 19.5% water and 0.5% formic acid) at a flowrate of 300 nL/min. Mass spectra were acquired in datadependent acquisition mode using a Top12 method in the Orbitrap analyzer with a mass range of 300-1759 *m*/*z* at a resolution of 120,000 FWHM, maximum IT of 55 ms, and a target value of 3 × 10⁶ ions. Precursors were selected with an isolation window of 1.2 *m*/*z*. HCD fragmentation was performed at a normalized collision energy of 25. MS/MS spectra were acquired with a target value of 5 × 10⁴ ions at a resolution of 15,000 FWHM, maximum IT of 150 ms and a fixed first mass of 100 *m*/*z*. Peptides with a charge of +1, >6, or with unassigned charge state were excluded from fragmentation for MS², and dynamic exclusion for 30 s prevented repeated selection of precursors. Raw data were processed using MaxQuant software v1.6.3.4. MS/MS spectra were searched with the Andromeda search engine against the human UniProt database (15 May 2019) and were supplemented with sequences of the corresponding protein constructs of interest. Sequences of 248 common contaminant proteins and decoy sequences were automatically added during the search. Trypsin specificity was required and a maximum of two missed cleavages was allowed. Minimal peptide length was set to seven amino acids. Carbamidomethylation of cysteine residues was set as a fixed modification, whereas oxidation of methionine and protein N-terminal acetylation were set as variable modifications. MaxLFQ with ratio count 1 was enabled. Peptide-spectrum matches and proteins were retained if they were below a false discovery rate of 1%. Match between runs was enabled. Data were analyzed in Rv3.5.3 combined with RStudio v1.1.463. The LFQ intensities were log2 transformed and reverse and contaminant hits removed. LIMMA V3.40.2 was used for differential expression analysis based on those values.
**Mace Analysis:** MCF-7 and MCF-7_{NtFT4} cells were treated with 40 µg/mL insulin in serum-free medium for 24 h. Cells were harvested and RNA was isolated using the NucleoSpin RNA kit (Macherey-Nagel). The isolated RNA was digested with TURBO DNase and analyzed by agarose gel electrophoresis. Massive Analysis of cDNA Ends (MACE), including sample processing, quality control, sequencing with 1 × 10⁶ raw reads per sample and data analysis, was carried out by GenXPro. The average raw count of each gene within a library was divided by the geometric mean of all counts in all samples and the median of the quotients was calculated per library. Each raw count was then divided by the libraryspecific median value. FDR values were calculated according to Benjamini-Hochberg, and p-values were calculated using the DEseq R package. For downstream data analysis, thresholds for genes included in differential gene expression and GO enrichment analysis between starved and insulin-exposed cells and between the two cell lines were set to a FDR < 0.05 and a normalized count > 0.1. For GO enrichment analysis, the PANTHER classification system was used. Interaction networks were analyzed using STRING v11 (*62*) and only genes with a log₂ fold change > 1 or < -1, p < 0.001, and a mean value of normalized reads between the two samples > 5 were included.
**Transfection of suspension cultures:** For transfection, HEK-293T and HEK-293T_{NtFT4} cells were collected from 40 mL cultures and resuspended in 2 mL complete Ex-CELL 293 medium at a density of 2 × 10⁷ cells/mL in six-well plates (*42*). Transfection efficiency was tested using 10-30 µg/mL DNA and 30-90 µg/mL 25 kDa linear polyethylenimine (PEI, Polysciences). Plasmid DNA was added to the cells followed by PEI, and cells were incubated for 4 h before dilution to 1 × 10⁶ cells/mL in 40 mL complete medium. Transfection efficiency was determined by measuring the expression of pcDNA3-LifeAct-mRFP in single cells using a FACSCelesta device (BD Biosciences) after 24, 48, 72 and 96 h (excitation = 561 nm, detection = 610/20 nm). Protein yields after transient transfection were determined by transfecting cells with pAPT-1E4-IgG1 and collecting secreted protein from the medium supernatants for up to 10 days. The expression of 1E4-IgG1 was measured by enzyme-linked immunosorbent assay (ELISA) or by western blotting using anti-human κ light chain IgGAP and bevacizumab/Avastin (kindly provided by Dr. Nicole Raven) as a control. Assembly of the antibody heavy and light chains was confirmed by electrophoresis using reducing and non-reducing loading buffers.
**DAS-ELISA:** For the quantification of human IgGi, 96-well plates were coated with anti-human IgG, Fc specific (Sigma-Aldrich, #12136) in 50 mM carbonate buffer (pH 9.6) overnight at 4 °C. The following steps include intermediate washes with PBS + 0.1% Tween-20. Wells were blocked with PBS containing 5% nonfat milk for 1 h before diluted samples and standards (Avastin) were loaded and incubated for 2 h. The antibody was detected by incubation for 2 h with an anti-human kappa light chain-peroxidase secondary antibody (Sigma-Aldrich, #A7164) in blocking buffer, and subsequent staining with 3,3',5,5'-tetramethylbenzidine (TMB). The staining reaction was stopped with 1 M HCl and adsorption was measured at 450 nm using an Infinite 200 Pro plate reader (Tecan). All experiments were carried out as technical triplicates.
**Statistical analysis:** All boxplots in the figures were prepared using the default settings of OriginPro2020 (center line = median; box limits = upper and lower quartiles; whiskers = 1.5× interquartile range; points = outliers). Statistical analysis, if not stated otherwise, was carried out using OriginPro2020 v9.7.5.184 (OriginLab). Equality of variances was analyzed by one-way ANOVA and Levene's test, and pairwise comparisons were carried out using Welch's t-test. FDRs (MACE and LC-MS/MS analysis) were calculated according to Benjamini-Hochberg.

## Claims

1. **A recombinant vertebrate cell,** comprising (i) a nucleic acid sequence encoding for a non-vertebrate and/or artificial phosphatidylethanolamine-binding protein (PEBP) and/or (ii) a protein consisting of an amino acid sequence of the non-vertebrate and/or artificial phosphatidylethanolamine-binding protein (PEBP).

2. The recombinant vertebrate cell of claim 1, wherein the vertebrate cell is a mammalian cell, such as a human cell, a mouse cell or a hamster cell.

3. The recombinant vertebrate cell of claim 1 or 2, wherein the vertebrate and/or artificial PEBP is a plant- or insect PEBP.

4. The recombinant vertebrate cell of any one of claims 1 to 3, wherein the vertebrate and/or artificial PEBP consists of an amino acid sequence that when aligned with the sequence of SEQ ID NO: 1 (human PEBP1) does not comprise the most C terminal alpha helix, preferably does not comprise the sequence of amino acids 177 to 187 if SEQ ID NO: 1, nor a sequence that is 90% identical to such a sequence.

5. The recombinant vertebrate cell of any one 0 claims 1 to 4, wherein the vertebrate and/or artificial PEBP is a protein that is heterologous to the species of the vertebrate cell, and/or wherein the nucleic acid sequence is for a heterologous expression of the vertebrate and/or artificial PEBP.

6. The recombinant vertebrate cell of any one of claims 1 to 5, wherein the vertebrate cell expressing the vertebrate and/or artificial PEBP compared to the same vertebrate cell not expressing the vertebrate and/or artificial PEBP has a significantly increased vitality, growth and/or proliferation.

7. The recombinant vertebrate cell of any one of claims 1 to 6, wherein the non-vertebrate and/or artificial PEBP comprises, and preferably consists of, an amino acid sequence having at least 80% sequence identity to a sequence shown in SEQ ID NO: 2 (CG10298), SEQ ID NO: 3 (CG18594) or SEQ ID NO: 4: (NtFT4).

8. **A method producing a cell line with improved characteristics selected from cell vitality, cellular protein expression and cell growth,** the method comprising the steps of providing a candidate vertebrate cell of a selected vertebrate species, introducing into the vertebrate cell a non-vertebrate and/or artificial phosphatidylethanolamine-binding protein (PEBP) to obtain a cell with improved characteristics, and culturing the cell with improved characteristics to obtain the cell line with improved characteristic.

9. The method of claim 8, further comprising a step of selection of one cell clone with improved characteristics, and subsequence culturing to obtain a clonal cell-line with improved characteristic.

10. The method of claim 8 or 9, wherein the candidate vertebrate cell is a mammalian cell such as a human cell, a mouse cell or a hamster cell.

11. The method of any one of claims 8 to 10, wherein the vertebrate and/or artificial PEBP consists of an amino acid sequence that when aligned with the sequence of SEQ ID NO: 1 (human PEBP1) does not comprise the most C terminal alpha helix, preferably does not comprise the sequence of amino acids 177 to 187 if SEQ ID NO: 1, nor a sequence that is 90% identical to such a sequence.

12. **An isolated nucleic acid construct suitable for recombinant expression in a vertebrate cell or cell line,** comprising a coding element operatively linked with one or more expression elements which is suitable for regulating protein expression in the vertebrate cell, wherein the coding element comprises a nucleic acid sequence encoding a non-vertebrate and/or artificial phosphatidylethanolamine-binding protein (PEBP).

13. The isolated nucleic acid construct of claim 12, suitable for transient or stable transfection of the vertebrate cell.

14. **A recombinant vertebrate cell** comprising the nucleic acid construct of claim 12 or 13.

15. **A method for recombinant expression of a target protein,** comprising a step of culturing under suitable conditions a recombinant vertebrate cell of any one of the preceding claims or a cell line with improved characteristics of any one of the preceding claims, wherein the recombinant vertebrate cell or cell line with improved characteristics further comprises an expression construct encoding the target protein.
